(19) Europäisches Patentamt
European Patent Office
Office européen des brevets

(11) Publication number: 0 323 740 A2

(12) # EUROPEAN PATENT APPLICATION

(21) Application number: 88312221.0

(51) Int. Cl.[4]: C07K 7/00 , A61K 37/02

(22) Date of filing: 22.12.88

(30) Priority: 24.12.87 US 138893
26.08.88 US 237299
16.12.88 US 285916

(43) Date of publication of application:
12.07.89 Bulletin 89/28

(84) Designated Contracting States:
AT BE CH DE ES FR GB GR IT LI LU NL SE

(71) Applicant: CALIFORNIA BIOTECHNOLOGY, INC.
2450 Bayshore Parkway
Mountain View, CA 94043(US)

(72) Inventor: Scarborough, Robert M.
29831 Clearbrook Circle, No 2
Hayward California 94544(US)
Inventor: Lewicki, John A.
4465 Borina Drive
San Jose California 95129(US)
Inventor: Johnson, Lorin K.
4979 Dolores Drive
Pleasanton California 94566(US)

(74) Representative: Harrison, David Christopher et al
MEWBURN ELLIS & CO 2/3 Cursitor Street
London EC4A 1BQ(GB)

(54) **Linear analogs of atrial natriuretic peptides.**

(57) Compounds and compositions comprising synthetic analogs of Atrial Natriuretic Peptides are provided, together with methods for their production and use as natriuretics, diuretics and/or vasodilators, or as intermediates for or modulators of such useful compounds or of native Atrial Natriuretic Peptides.

EP 0 323 740 A2

# LINEAR ANALOGS OF ATRIAL NATRIURETIC PEPTIDES

## Technical Field

The invention relates to the field of metabolic regulation of the cardiovascular system. In particular, it is directed to classes of compounds with natriuretic, diuretic, and/or vasorelaxant activities.

## Background Art

The published PCT application of the Applicants herein, WO87/02674 published 7 May 1987 and incorporated herein by reference, describes a class of vasoactive peptides which are similar to those found in atrial tissue and responsible for regulation of the tension in the cardiovascular system. These compounds are synthetic linear analogs of the cyclic native atrial natriuretic peptides (ANP). Besides disclosure of a specific class of linear peptides which are useful for their natriuretic, diuretic, and/or vasorelaxant activities, the published application describes methods to synthesize these peptides as well as to assess their ability to regulate salt and water balance and cardiovascular tension in vivo.

As disclosed in the published application, linear synthetic forms of the atrial peptides can be synthesized wherein the N-terminus is supplemented with a non-peptide group which is generally hydrophobic in nature; Examples 306 and 307 of that application describe the means to synthesize such analogs.

Furthermore, the published application describes biological assays which can, in simple in vitro tests, predict the ability of the tested compounds to behave as cardiovascular regulators in vivo. The receptor-binding assays, using competition with native ANP for binding to cultured BASM or BAE cells, was demonstrated to correlate with the results of whole mammal assays in anesthetized rats and dogs. In isolated tissue assays, the compounds, although active in vivo, were generally not active (for instance in isolated perfused rat kidney). Nevertheless, they were able to potentiate the effect of native ANP in these isolated tissues. The results in both the in vivo and in vitro assay indicated, although no particular theory is binding on Applicants, that the synthetic analogs of the invention may be effective, at least in part, due to binding of clearance receptors for native ANP.

The experimental results in the published application are cited and incorporated herein by reference.

## Disclosure of the Invention

Most of the synthetic analog compounds of the present invention retain a core pentapeptide sequence of amino acid residues which correspond in a defined way to the sequence $AA_8$-$AA_{12}$ of native ANPs, using the identification system from Atlas, S., et al., Nature (1984) 309:171-719 wherein the amino-terminal arginine residue is at position 1. In the known native ANPs, this core sequence is RIDRI in rat and RMDRI in human. Certain defined permutations of this sequence, including some wherein $AA_{12}$ is not present, retain activity in vivo and demonstrate that the core peptide structure is a significant factor in the peptides' biological activity. However, as explained herein, many of these compounds are not active in in vitro model systems for assay of diuretic or natriuretic activities. It is likely that these analogs empower the function of endogenous ANPs by blocking clearance receptor(s) for these peptides.

The present invention is, therefore, in one aspect directed to linear analog peptide compounds, having natriuretic, diuretic and/or vasorelaxant activity in mammals, which have the formula:

$Z_1Z_2$-$AA_8$-$AA_9$-$AA_{10}$-$AA_{11}$-$AA_{12}$-$Z_3$ (1)

wherein:

each of $AA_8$ and $AA_{11}$ is, independently, preferably a basic/noncyclic, but can be also a neutral/nonpolar/small or neutral/polar/large/nonaromatic amino acid residue; in addition, $AA_8$ can be a neutral/nonpolar/large/nonaromatic amino acid;

$AA_9$ is a neutral/nonpolar/large/nonaromatic amino acid residue in the D or L configuration;

$AA_{10}$ is an acidic amino acid residue; and

$AA_{12}$ is a neutral/nonpolar/large/nonaromatic amino acid residue, in the D or L configuration or is a covalent bond;

wherein $Z_1$ is a peptide of from 1 to 125 amino acids having as its carboxy-terminal residue a hydrophobic amino acid residue, or the $desNH_2$ form thereof, or is a hydrophobic aliphatic, aromatic, or mixed

aliphatic/aromatic organic group of from 6 to 20 carbon atoms,
$Z_2$ is a spacer group which provides a spaced dimension of about 4.5-15 angstroms, i.e., contains 3-9 atoms in a linked group or can be conformed to the proper spacing by folding; and
$Z_3$ is (OH), $NH_2$, NHR′ or NR′R″ wherein R′ or R″ are each independently straight or branched chain alkyl of 1-10 carbon atoms wherein 1 or 2 carbons may be replaced by O, N, or S, or $Z_3$ is a peptide residue of 1-20 amino acid residues, or an amide or alkyl amide thereof, with the proviso that when $AA_{12}$ is a covalent bond, $Z_3$ cannot be OH, $NH_2$ or a peptide, wherein
one or more of the amide backbone linkages between any adjacent amino acid residues is replaced by a linkage selected from the group consisting of $-CH_2NH-$, $-CH_2-S-$, $-CH_2CH_2-$, -CH=CH- (cis and trans), $-COCH_2-$, $-CH(OH)CH_2$ and $-CH_2SO-$.

In another aspect, the invention is directed to linear analog peptide compounds, having natriuretic, diuretic and/or vasorelaxant activity in mammals, which have the formula:
$Z_1Z_2$-$AA_8$-$AA_9$-$AA_{10}$-$AA_{11}$-$AA_{12}$-$Z_3$ (1)
wherein:
each of $AA_8$ and $AA_{11}$ is, independently, preferably a basic/noncyclic, but can be also a neutral/nonpolar/small or neutral/polar/large/nonaromatic amino acid residue; in addition, $AA_8$ can be a neutral/nonpolar/large/nonaromatic amino acid;
$AA_9$ is a neutral/nonpolar/large/nonaromatic amino acid residue in the D or L configuration;
$AA_{10}$ is an acidic amino acid residue; and
$AA_{12}$ is a covalent bond;
wherein $Z_1$ is a peptide of from 1 to 125 amino acids having as its carboxy-terminal residue a hydrophobic amino acid residue, or the desNH2 form thereof, or is a hydrophobic aliphatic, aromatic, or mixed aliphatic/aromatic organic group of from 6 to 20 carbon atoms,
$Z_2$ is a spacer group which provides a spaced dimension of about 4.5-15 angstroms, i.e., contains 3-9 atoms in a linked group or can be conformed to the proper spacing by folding; and
$Z_3$ is NHR′ or NR′R″ wherein R′ or R″ are each independently straight or branched chain alkyl of 1-10 carbon atoms wherein 1 or 2 carbons may be replaced by O, N, or S.

In the foregoing compounds of the invention, one or more of the amide backbone linkages between any adjacent amino acid residues may optionally be replaced by a linkage selected from the group consisting of $-CH_2NH$, $-CH_2-S-$, $-CH_2CH_2-$, -CH=CH- (cis and trans), $-COCH_2-$, $-CH(OH)CH_2$ and $-CH_2SO-$.

In still another aspect, the invention is directed to linear analog peptide compounds, having natriuretic, diuretic and/or vasorelaxant activity in mammals, which have the formula:
$Z_1Z_2$-$AA_8$-$AA_9$-$AA_{10}$-$AA_{11}$-$AA_{12}$-$Z_3$ (1)
wherein:
each of $AA_8$ and $AA_{11}$ is, independently, preferably a basic/noncyclic, but can be also a neutral/nonpolar/small or neutral/polar/large/nonaromatic amino acid residue; in addition, $AA_8$ can be a neutral/nonpolar/large/nonaromatic amino acid;
$AA_9$ is a neutral/nonpolar/large/nonaromatic amino acid residue in the D or L configuration;
$AA_{10}$ is an acidic amino acid residue; and
$AA_{12}$ is a neutral/nonpolar/large/nonaromatic amino acid residue, in the D or L configuration or is a covalent bond;
wherein $Z_1$ is a peptide of from 1 to 125 amino acids having as its carboxy-terminal residue a hydrophobic amino acid residue, or the desNH2 form thereof, or is a hydrophobic aliphatic, aromatic, or mixed aliphatic/aromatic organic group of from 6 to 20 carbon atoms,
$Z_2$ is a spacer group which provides a spaced dimension of about 4.5-15 angstroms, i.e., contains 3-9 atoms in a linked group or can be conformed to the proper spacing by folding and is selected from the group consisting of:
$-(P)_n-(CO)_x-$ wherein x is 0 or 1, n is 1-6, and P is $CH_2$, wherein 1-2 of said $-CH_2-$ groups can be replaced by NH, provided N-N does not occur, with the proviso that $Z_2$ cannot be $-NH(CH_2)_{3-6}CO-$; and
$-(Q)_m-B-(Q)_m-(CO)_x-$wherein x is 0 or 1, each m is independently 0-3 but the sum of both m is 5 or less; Q is $CH_2$ or NH, with the proviso that -N-N- does not occur, and B is a saturated or unsaturated five- or six-membered ring optionally containing an N heteroatom and B can be joined to Q either 1,4 or 1,3;
$Z_3$ is (OH), $NH_2$, NHR′ or NR′R″ wherein R′ or R″ are each independently straight or branched chain alkyl of 1-10 carbon atoms wherein 1 or 2 carbons may be replaced by O, N, or S, or $Z_3$ is a peptide residue of 1-20 amino acid residues, or an amide or alkyl amide thereof, with the proviso that when $AA_{12}$ is a covalent bond, $Z_3$ cannot be OH, $NH_2$ or a peptide.

In the foregoing compounds of the invention, one or more of the amide backbone linkages between any adjacent amino acid residues may optionally be replaced by a linkage selected from the group consisting of

$-CH_2NH-$, $-CH_2-S-$, $-CH_2CH_2-$, $-CH=CH-$ (cis and trans), $-COCH_2-$, $-CH(OH)CH_2$ and $-CH_2SO-$.

In still another aspect, the invention is directed to linear analog peptide compounds, having natriuretic, diuretic and/or vasorelaxant activity in mammals, which have the formula:

$Z_1Z_2-AA_8-AA_9-AA_{10}-AA_{11}-AA_{12}-Z_3$ (1)

wherein:

each of $AA_8$ and $AA_{11}$ is, independently, preferably a basic-noncyclic, but can be also a neutral/nonpolar/small or neutral/polar/large/nonaromatic amino acid residue; in addition, $AA_8$ can be a neutral/nonpolar/large/nonaromatic amino acid;

$AA_9$ is a neutral/nonpolar/large/nonaromatic amino acid residue in the D or L configuration;

$AA_{10}$ is an acidic amino acid residue; and

$AA_{12}$ is a neutral/nonpolar/large/nonaromatic amino acid residue, in the D or L configuration or is a covalent bond;

wherein $Z_1$ is of the formula $R'_1CO$, $R'_1COCH_2$, $R'_1O$- or $R'_1$, wherein $R'_1$ contains at least one halo group alpha to a carbonyl moiety;

$Z_2$ is a spacer group which provides a spaced dimension of about 4.5-15 angstroms, i.e., contains 3-9 atoms in a linked group or can be conformed to the proper spacing by folding; and

$Z_3$ is (OH), $NH_2$, $NHR'$ or $NR'R''$ wherein $R'$ or $R''$ are each inependently straight or branched chain alkyl of 1-10 carbon atoms wherein 1 or 2 carbons may be replaced by O, N, or S, or $Z_3$ is a peptide residue of 1-20 amino acid residues, or an amide or alkyl amide thereof, with the proviso that when $AA_{12}$ is a covalent bond, $Z_3$ cannot be OH, $NH_2$ or a peptide.

In the foregoing compounds of the invention, one or more of the amide backbone linkages between any adjacent amino acid residues may optionally be replaced by a linkage selected from the group consisting of $-CH_2NH-$, $-CH_2-S-$, $-CH_2CH_2-$, $-CH=CH-$ (cis and trans), $-COCH_2-$, $-CH(OH)CH_2$ and $-CH_2SO-$.

In the peptides of all of the various aspects of the invention, one or two of the amino acid residues may be replaced by the corresponding D isomer, in addition to, or instead of, $AA_9$ and, if applicable, $AA_{12}$.

The invention is also directed to pharmaceutical compositions useful as natriuretics, diuretics, vasodilators and/or modulators of the renin-angiotensinaldosterone system, which compositions containing the above-recited analog peptide compounds, including their amides and esters, and the nontoxic addition salts thereof, together with a pharmaceutically acceptable liquid, gel or solid carrier. Administration of therapeutically effective doses of these compositions can provide effective delivery of the above-recited biological activities to mammalian hosts.

Additional aspects of the present invention provide methods for producing such compounds and compositions, and methods for using the compounds and compositions as therapeutic agents.

## Brief Description of the Drawings

Figure 1 schematically outlines the classification of amino acids as used herein.

Figure 2 gives a list of various compounds of the invention.

Figure 3 gives a list of various compounds which are similar to those of the invention, but which are outside its scope.

## Modes of Carrying Out the Invention

In accordance with the present invention, several classes of novel analogs of native Atrial Natriuretic Peptide (ANP) compounds are provided wherein these analogs are capable of exhibiting or modulating the natriuretic, diuretic and/or vasorelaxant activity of the native peptides in mammals in vivo.

The sequence of amino acid residues of the present synthetic analog compounds, including the core pentapeptide, and preferred embodiments thereof, are defined in terms of amino acids of certain characteristics of particular subclasses.

Amino acid residues can be generally subclassified into four major subclasses as follows and as shown in Figure 1.

Acidic: The residue has a negative charge due to loss of H ion at physiological pH and the residue is attracted by aqueous solution so as to seek the surface positions in the conformation of a peptide in which it is contained when the peptide is in aqueous medium at physiological pH.

Basic: The residue has a positive charge due to association with H ion at physiological pH and the residue is attracted by aqueous solution so as to seek the surface positions in the conformation of a peptide

in which it is contained when the peptide is in aqueous medium at physiological pH.

Neutral/nonpolar: The residues are not charged at physiological pH and the residue is repelled by aqueous solution so as to seek the inner positions in the conformation of a peptide in which it is contained when the peptide is in aqueous medium. These residues are also designated "hydrophobic" herein.

Neutral/polar: The residues are not charged at physiological pH, but the residue is attracted by aqueous solution so as to seek the outer positions in the conformation of a peptide in which it is contained when the peptide is in aqueous medium.

It is understood, of course, that in a statistical collection of individual residue molecules some molecules will be charged, and some not, and there will be an attraction for or repulsion from an aqueous medium to a greater or lesser extent. To fit the definition of "charged", a significant percentage (at least approximately 25%) of the individual molecules are charged at physiological pH. The degree of attraction or repulsion required for classification as polar or nonpolar is arbitrary, and, therefore, amino acids specifically contemplated by the invention have been specifically classified as one or the other. Most amino acids not specifically named can be classified on the basis of known behavior.

Amino acid residues can be further subclassified as cyclic or noncyclic, and aromatic or nonaromatic, self-explanatory classifications with respect to the side chain substituent groups of the residues, and as small or large. The residue is considered small if it contains a total of 4 carbon atoms or less, inclusive of the carboxyl carbon. Small residues are, of course, always nonaromatic.

For the naturally occurring protein amino acids, subclassification according to the foregoing scheme is as follows (see also Figure 1).

<u>Acidic</u>: Aspartic acid and Glutamic acid;
<u>Basic/noncyclic</u>: Arginine, Lysine;
<u>Basic/cyclic</u>: Histidine;
<u>Neutral/polar/small</u>: Glycine, Serine and Cysteine;
<u>Neutral/polar/large/nonaromatic</u>: Threonine, Asparagine, Glutamine;
<u>Neutral/polar/large/aromatic</u>: Tyrosine;
<u>Neutral/nonpolar/small</u>: Alanine;
<u>Neutral/nonpolar/large/nonaromatic</u>: Valine, Isoleucine, Leucine, Methionine;
<u>Neutral/nonpolar/large/aromatic</u>: Phenylalanine, and Tryptophan.

The gene-encoded amino acid proline, although technically within the group neutral/nonpolar/large/cyclic and nonaromatic, is a special case due to its known effects on the secondary conformation of peptide chains, and is not, therefore, included in this defined group.

Certain commonly encountered amino acids, which are not encoded by the genetic code, include, for example, beta-alanine (beta-ala), or other omega-amino acids, such as 3-amino proprionic, 4-amino butyric and so forth, alpha-aminoisobutyric acid (Aib), sarcosine (Sar), ornithine (Orn), citrulline (Cit), t-butylalanine (t-BuA), t-butylglycine (t-BuG), N-methylisoleucine (N-MeIle), phenylglycine (Phg), and cyclohexylalanine (Cha), norleucine (Nle), cysteic acid (Cya) and methionine sulfoxide (MSO). These also fall conveniently into particular categories.

Based on the above definition,
Sar and beta-ala are neutral/nonpolar/small;
t-BuA, t-BuG, N-MeIle, Nle and Cha are neutral/nonpolar/large/nonaromatic;
Orn is basic/noncyclic;
Cya is acidic;
Cit, Acetyl Lys, and MSO are neutral/polar/large/nonaromatic; and
Phg is neutral/nonpolar/large/aromatic.

See, also, Figure 1.

The various omega-amino acids are classified according to size as neutral/nonpolar/small (beta-ala, i.e., 3-aminopropionic, 4-aminobutyric) or large (all others).

Other amino acid substitutions for those encoded in the gene can also be included in peptide compounds within the scope of the invention and can be classified within this general scheme.

The nomenclature used to describe ANP analog compounds of the present invention follows the conventional practice wherein the amino group is assumed to the left and the carboxy group to the right of each amino acid in the peptide. In the formulas representing selected specific embodiments of the present invention, the amino- and carboxy-terminal groups, although often not specifically shown, will be understood to be in the form they would assume at physiological pH values, unless otherwise specified. Thus, the N-terminal $H^{+}_{2}$ and C- terminal-$O^{-}$ at physiological pH are understood to be present through not necessarily

specififed and shown, either in specific examples or in generic formulas. In the peptides shown, each encoded residue where appropriate is represented by a single letter designation, corresponding to the trivial name of the amino acid, in accordance with the following conventional list:

| Amino Acid | One-Letter Symbol |
|---|---|
| Alanine | A |
| Arginine | R |
| Asparagine | N |
| Aspartic acid | D |
| Cysteine | C |
| Glutamine | Q |
| Glutamic acid | E |
| Glycine | G |
| Histidine | H |
| Isoleucine | I |
| Leucine | L |
| Lysine | K |
| Methionine | M |
| Phenylalanine | F |
| Proline | P |
| Serine | S |
| Threonine | T |
| Tryptophan | W |
| Tyrosine | Y |
| Valine | V |

The amino acids not encoded genetically are abbreviated as indicated above.

In the specific peptides shown in the present application, the L-form of any amino acid residue having an optical isomer is intended unless otherwise expressly indicated by a dagger superscript (†). While the residues of the invention peptides are normally in the natural L optical isomer form, one or two, preferably one, amino acid in addition to as well as instead of $AA_9$ and/or $AA_{12}$, may be replaced with the optical isomer D form (including embodiments where $AA_9$ and $AA_{12}$ are both L).

Free functional groups, including those at the carboxy- or amino-terminus, can also be modified by amidation, acylation or other substitution, where can, for example, change the solubility of the compounds without affecting their activity.

In particular, it has been discovered that carboxy terminal amide-modified analogs of Atrial Natriuretic Peptides are particularly potent and therefore preferred embodiments of the present invention. In general, the nitrogen atom of the amido group, covalently bound to the carbonyl carbon, will be $NH_2$, $-NHR'$, or $NR'R''$, wherein $R'$ and $R''$ are straight or branched chain alkyl or alkyl acyl of 1-10C, preferably 1-6C, including these groups wherein 1-2 carbons are replaced by nitrogen, oxygen or sulfur atoms. Representatives of such amido groups are: $-NH_2$, $-NHCH_3$, $-N(CH_3)_2$, $-NHCH_2CH_3$, $-NHC_6H_5$, $-NHCH_2CH(CH_3)_2$, $-NHCH_2CH(CH_3)CH_2CH_3$, $-NHCH_2CH_2OH$, $-NHCH_2-OCH_2CH_3$ and $-N(CH_3)CH_2CH_2SCH_2CH_3$, among others.

In forming amidated analogs of the present invention, the analog compounds can be synthesized directly, for example using Boc-$AA_x$-pMBHA-Resin or Boc-$AA_x$-BHA-Resin, wherein $AA_x$ is the selected carboxy-terminal amino acid of the desired analog compound as described in further detail below. Alternatively, the analog compounds of the present invention can be chemically or enzymatically amidated subsequent to peptide synthesis using means well known to the art, or prepared by standard solution-phase peptide synthesis protocols.

## Preferred Embodiments

### A. The Core Pentapeptide

The compounds of the invention all contain the pentapeptide core sequence:
$AA_8$-$AA_9$-$AA_{10}$-$AA_{11}$-$AA_{12}$,
wherein each of $AA_8$ and $AA_{11}$ is, independently:
a basic/noncyclic; or
a neutral/nonpolar/small; or
a neutral/polar/large/nonaromatic amino acid residue;
in addition, $AA_8$ can be a neutral/nonpolar/large/nonaromatic amino acid;
$AA_9$ is a neutral/nonpolar/nonaromatic amino acid residue in the D or L configuration;
$AA_{10}$ is an acidic amino acid residue; and
$AA_{12}$ is a neutral/nonpolar/large/nonaromatic amino acid residue in the D or L configuration, or is a covalent bond.

The most preferred sequence of this core is R(I/M)DRI, wherein all residues are in the L configuration and the amino acid residues contained within the parentheses are alternatives. Next in preference are those sequences wherein only one of the R(I/M)DRI residues has been substituted by an alternative residue within the above definitions. Preferred substitutions are:
For $AA_8$, instead of R: K, Acetyl Lys, A, Q, N, L or Nle;
for $AA_9$, instead of I/M: V, V†, L, L†, I†, M†, t-BuA, t-BuG, or Cha;
for $A_{10}$, instead of D: E or Cya;
for $A_{11}$, instead of R: K. Acetyl Lys, A, Q, N, Orn, or Cit;
for $A_{12}$, instead of I: M, M†, V, V†, L, L†, I†, N-MeIle, t-BuA, or a covalent bond.

Particularly preferred are those embodiments wherein this sequence is selected from the group consisting of:

| | | |
|---|---|---|
| A(I/M)DRI<br>K(I/M)DRI | RM†DRI<br>RLDRI | R(I/M)DRL<br>R(I/M)DRM |
| Acetyl Lys (I/M)DRI | | |
| Q(I/M)DRI<br>RVDRI<br>RI†DRI | R(I/M)ERI<br>R(I/M)DKI<br>R(I/M)DQI | R(I/M)DRM†<br>R(I/M)DRI†<br>R(I/M)DRV |

More than one alteration from the naturally occurring RIDRI or RMDRI sequence is within the scope of the invention, but less preferred. Particularly favored subsets of this group include those wherein glutamic replaceds aspartic as $AA_{10}$, in addition to another substitution.

## B. Embodiments of $Z_1$

Preferred forms of $Z_1$ include in addition to non-amino acid hydrophobic residues, described below, peptides of 1-5, more usually 1-3, amino acids or the $desNH_2$ forms thereof, wherein the C-terminal amino acid is hydrophobic, i.e. neutral and nonpolar, and most particularly is Phe or $desNH_2$-Phe. Preferred embodiments of $Z_1$ include R-S-S-C-F, R-S-S-A-F, Y-A-F, R-C-F, S-C-F, A-F, C-F, and F or $desNH_2$-F, i.e., those wherein the C-terminal end amino acid residue is F, and the upstream residues are selected from Y, A, C, S, and R.

Preferred nonpeptide-derived forms of $Z_1$ include organic substituent groups which are generally nontoxic, hydrophobic and relatively large or bulky when compared to substituent groups ordinarily found with amino acid residues.

One class of presently preferred organic substituent groups can be represented by the general formula:
$R_1$-CO-
wherein $R_1$ is an organic hydrophobic group. Included in this formula are 2-substituted acetyl, 3-substituted propionyl, and 4-substituted butyryl groups, wherein the substitutions to these groups include the general class of neutral, hydrophobic mono- and polycyclic aromatic or saturated ring systems. In embodiments wherein a halogen alpha to a CO is contained, the substituent is designated $R'_1$CO. Other classes have the general formulas $R_1$-$COCH_2$-, $R_1$-0-, $R'_1$-$COCH_2$, $R'_1$-O-, or simply $R_1$- or $R'_1$. Representative examples of the preferred substituent groups include:

fluorenylmethyloxycarbonyl (FMOC)

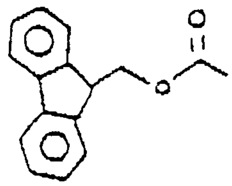

benzyloxycarbonyl (CBZ)

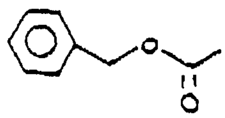

2-(2'-(6'-methoxynaphthyl)) propionyl
2-(2'-(6'-MeONAP)

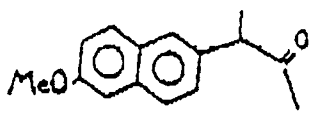

Diphenylpropionyl (DPP)

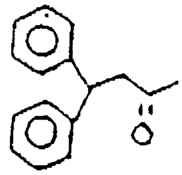

Biphenylacetyl (BPA)

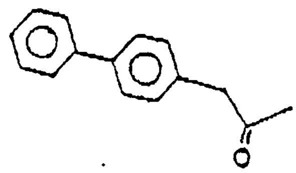

Triphenylpropionyl (TPP)

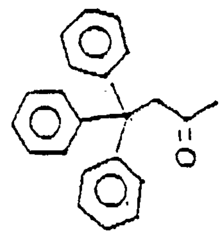

Cyclohexylacetyl (CHA)

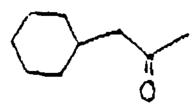

3-Indolepropionyl (3-1P)

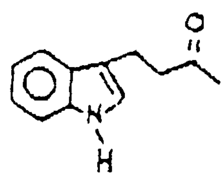

4-Indolebutyryl (4-IB)

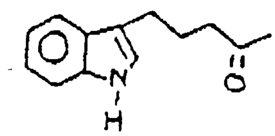

1-Adamantylacetyl (AA)

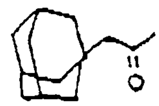

1-Naphthylacetyl (1-NA)

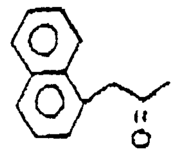

2-Naphthylacetyl (2-NA)

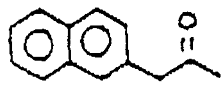

1-Naphthoxyacetyl (1-NOA)

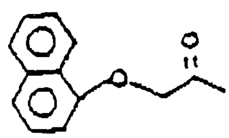

2-Naphthoxyacetyl (2-NOA)

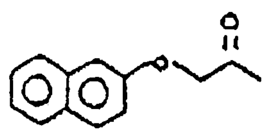

Dibenzylacetyl (DBA)

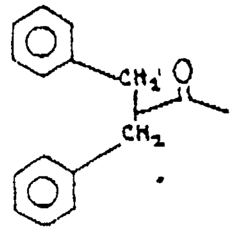

bis (1′-naphthylmethyl) acetyl (BNMA)

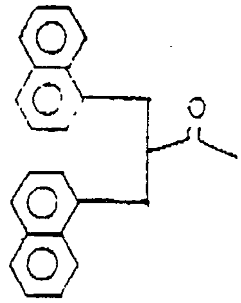

2-naphthylthioacetyl (2-NTA)

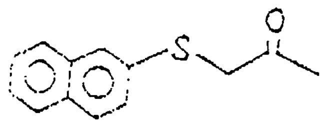

3-phenoxypropionyl (3-POP)

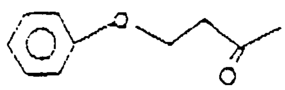

2-Naphthoyl (2-NYL)

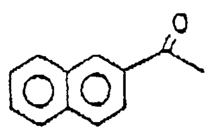

2-naphtoxy (2-NO)

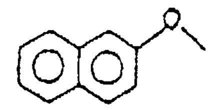

2-naphthyl (2-NL)

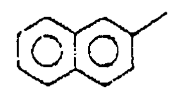

2-halo acetyl phenylalanyl, e.g. 2ClAF or 2BrAF
$ClCH_2CO$-F- or $BrCH_2CO$-F-
2-halo phenylacetyl, e.g., 2ClPA or 2BrPA

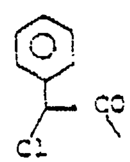

or

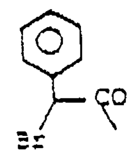

groups. Particularly preferred are NA, NOA, and NYL.

The 2-halo forms are thought to have the possibility to bind covalently to the clearance receptor as "suicide" binders.

## C. Embodiments of $Z_2$

In the compounds of the invention, $Z_2$ provides a spacer element separating $AA_8$ from the hydrophobic portion of $Z_1$. The linker $Z_2$ must be capable to achieve a distance between $AA_8$ and the hydrophobe of about between 4.5 and 15 angstroms, corresponding to 3-9 atoms in a normally extended chain. Of course, longer linkers can be used provided their 3-dimensional conformations permit this spacing distance to be accommodated.

Preferred embodiments for $Z_2$ are selected from the group consisting of

(a) $(AA)_a$ wherein AA is an amino acid and a is 1 or 2, especially wherein each AA is selected from G, S, A, Sar, and Aib;

(b) $-(P)_n-(CO)_x-$ wherein x is 0 or 1, n is 1-6, and P is $CH_2$, wherein 1-2 of said $-CH_2-$ groups can be replaced by NH, provided N-N does not occur; and

(c) $-(Q)_m-B-(Q)_m-(CO)_x-$ wherein x is 0 or 1, each m is independently 0-3 but the sum of both m is 5 or less; Q is $CH_2$ or NH, with the proviso that -N-N- does not occur, and B is a saturated or unsaturated five- or six-membered ring optionally containing an N heteroatom. B can be joined to Q either 1, 4 or 1, 3. Particularly preferred is p-aminophenyl acetyl (4-APA).

## D. Embodiments of $Z_3$

Preferred for $Z_3$ are $NH_2$, $NHR'$, and the amide or alkyl amide of peptide residues of 1-3 amino acids, except for those compounds wherein $AA_{12}$ is a bond. When $AA_{12}$ is a covalent bond, $Z_3$ is preferred to be the alkyl amidated form, e.g., $-NHR'$ wherein $R'$ is 2-10C. Especially preferred among the embodiments which are peptide residues are those wherein the amino acids are selected from G, A, and S.

E. Non-Peptide Linkages

In one embodiment of the invention, the amide linkages (-CO-NH-) within the core pentapeptide or those described above within $Z_1$ and/or $Z_2$ and/or $Z_3$ can be replaced with other types of linkages such as $-CH_2NH-$, $-CH_2-S-$, $-CH_2CH_2-$, -CH=CH- (cis and trans), $-COCH_2-$, $-C(OH)CH_2-$ and $-CH_2SO-$, by methods known in the art. The following references describe preparation of peptide analogs which include these alternative-linking moieties: Spatol, A.F., Vega Data (March 1983) 1:3 "Peptide Backbone Modifications" (general review); Spatola, A.F. in "Chemistry and Biochemistry of Amino Acids Peptides and Proteins", 1983 (B. Weinstein et al., eds.) Marcel Dekker, New York, p. 267 (general review); Morley, J.S., Trends Pharm Sci (1980) pp. 463-468 (general review); Hudson, D. et al., Int J Pept Prot Res (1979) 14:177-185 ($-CH_2NH-$, $-CH_2CH_2-$); Spatola, A.F. et al., Life Sci (1986) 38:1243-1249 ($-CH_2-S$); Hann, M.M., J Chem Soc Perkin Trans I (1982) 307-314 (-CH-CH-, cis and trans); Almquist, R.G. et al., J. Med Chem (1980) 23:1392-1398 ($-COCH_2-$); Jennings-White, C. et al., Tetrahedron Lett (1982) 23:2533 ($-COCH_2-$); Szelke, M., et al., European Application No. EP 45665 (1982) CA: 97: 39405 ($-CH(OH)CH_2-$); Holladay, M.W. et al., Tetrahedron Lett (1983) 24:4401-4404 ($-C(OH)CH_2-$); and Hruby, V.J., Life Sci (1982) 31:189-199 ($-CH_2-S-$). Particularly preferred is $-CH_2NH-$.

Synthesis

Compounds within the scope of the present invention can be synthesized chemically by means well known in the art such as, e.g., solid-phase peptide synthesis. The synthesis is commenced from the carboxy-terminal end of the peptide using an alpha-amino protected amino acid. t-Butyloxycarbonyl (Boc) protective groups can be used for all amino groups even through other protective groups are suitable. For example, Boc-Asn-OH, Boc-Ser-OH, Boc-Phe- OH, Boc-Arg-OH or Boc-Tyr-OH (i.e., selected ANP analog carboxy-terminal amino acids) can be esterified to chloromethylated polystyrene resin supports. The polystyrene resin support is preferably a copolymer of styrene with about 0.5 to 2% divinyl benzene as a cross-linking agent which causes the polystyrene polymer to be completely insoluble in certain organic solvents. See Stewart et al., Solid-Phase Peptide Synthesis (1969) W.H. Freeman Co., San Francisco and Merrifield, J Am Chem Soc (1963) 85:2149-2154. These and other methods of peptide synthesis are also exemplified by US Patent Nos. 3,862,925, 3,842,067, 3,972,859, and 4,105,602.

The synthesis may use manual techniques or automatically employing, for example, an Applied BioSystems 430A Peptide Synthesizer (Foster City, California) or a Biosearch SAM II automatic peptide synthesizer (Biosearch, Inc. San Rafael, California), following the instructions provided in the instruction manual supplied by the manufacturer.

It will be readily appreciated by those having ordinary skill in the art of peptide synthesis that the intermediates which are constructed in accordance with the present disclosure during the course of synthesizing the present analog compounds are themselves novel and useful compounds and are thus within the scope of the invention.

Alternatively, selected compounds of the present invention can be produced by expression of recombinant DNA constructs prepared in accordance with well-known methods. Such production can be desirable to provide large quantities or alternative embodiments of such compounds. Since the peptide sequences are relatively short, recombinant production is facilitated.

Administration and Use

Compounds of the present invention are shown to have natriuretic, diuretic and hypotensive activity in the intact mammal, and may possess vasorelaxant activity or inhibit the release of aldosterone and renin.

Thus these compounds, and compositions containing them, can find use as therapeutic agents in the treatment of various edematous states such as, for example, congestive heart failure, nephrotic syndrome and hepatic cirrhosis, pulmonary disease, in addition to hypertension and renal failure due to ineffective renal perfusion or reduced glomerular filtration rate.

Thus the present invention also provides compositions containing an effective amount of compounds of the present invention, including the nontoxic addition salts, amides and esters thereof, which may, alone, serve to provide the above-recited therapeutic benefits. Such compositions can also be provided together with physiologically tolerable liquid, gel or solid diluents, adjuvants and excipients.

These compounds and compositions can be administered to mammals for veterinary use, such as with

domestic animals, and clinical use in humans in a manner similar to other therapeutic agents. In general, the dosage required for therapeutic efficacy will range from about 0.01 to 1000 mcg/kg, more usually 0.1 to 1000 mcg/kg of the host body weight. Alternatively, dosages within these ranges can be administered by constant infusion over an extended period of time until the desired therapeutic benefits have been obtained.

Typically, such compositions are prepared as injectables, either as liquid solutions or suspensions; solid forms suitable for solution in, or suspension in, liquid prior to injection may also be prepared. The preparation may also be emulsified. The active ingredient is often mixed with diluents or excipients which are physiologically tolerable and compatible with the active ingredient. Suitable diluents and excipients are, for example, water, saline, dextrose, glycerol, or the like, and combinations thereof. In addition, if desired the compositions may contain minor amounts of auxiliary substances such as wetting or emulsifying agents, stabilizing or pH-buffering agents, and the like.

The compositions are conventionally administered parenterally, by injection; for example, either subcutaneously or intravenously. Additional formulations which are suitable for other modes of administration include suppositories, intranasal aerosols, and, in some cases, oral formulations. For suppositories, traditional binders and excipients may include, for example, polyalkylene glycols or triglycerides; such suppositories may be formed from mixtures containing the active ingredient in the range of 0.5% to 10% preferably 1%-2%. Oral formulations include such normally employed excipients as, for example, pharmaceutical grades of mannitol, lactose, starch, magnesium stearate, sodium saccharin, cellulose, magnesium carbonate, and the like. These compositions take the form of solutions, suspensions, tablets, pills, capsules, sustained-release formulations, or powders, and contain 10%-95% of active ingredient, preferably 25%-70%.

The peptide compounds may be formulated into the compositions as neutral or salt forms. Pharmaceutically acceptable nontoxic salts include the acid addition salts (formed with the free amino groups) and which are formed with inorganic acids such as, for example, hydrochloric or phosphoric acids, or organic acids such as acetic, oxalic, tartaric, mandelic, and the like. Salts formed with the free carboxyl groups may be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

In addition to the compounds of the present invention which display natriuretic, diuretic or vasorelaxant activity, compounds of the present invention can also be employed as intermediates in the synthesis of such useful compounds. Alternatively, by appropriate selection, compounds of the present invention whose activity levels are reduced or eliminated entirely can serve to modulate the activity of other diuretic, natriuretic or vasorelaxant compounds, including compounds outside the scope of the present invention, by, for example, binding to alternate receptors, stimulating receptor turnover, or providing alternate substrates for degradative enzyme or receptor activity and thus inhibiting these enzymes or receptors. When employed in this manner, such compounds can be delivered as admixtures with other active compounds or can be delivered separately, for example, in their own carriers.

Compounds of the present invention can also be used for preparing antisera for use in immunoassays employing labeled reagents, usually antibodies. Conveniently, the polypeptides can be conjugated to an antigenicity-conferring carrier, if necessary, by means of dialdehydes, carbodiimide or using commercially available linkers. These compounds and immunologic reagents may be labeled with a variety of labels such as chromophores, fluorophores such as, e.g., fluorescein or rhodamine, radioisotopes such as $^{125}I$, $^{35}S$, $^{14}C$, or $^{3}H$, or magnetized particles, by means well known in the art.

These labeled compounds and reagents, or labeled reagents capable of recognizing and specifically binding to them, can find use as, e.g., diagnostic reagents. Samples derived from biological specimens can be assayed for the presence or amount of substances having a common antigenic determinant with compounds of the present invention. In addition, monoclonal antibodies can be prepared by methods known in the art, which antibodies can find therapeutic use, e.g., to neutralize overproduction of immunologically related compounds in vivo.

The following examples are provided by way of illustration, rather than implying any limitation of the subject invention.

## Examples

In the experimental disclosure which follows, the amino acid sequence of chemically synthesized ANP analog compounds are numbered from the amino-terminal arginine residue corresponding to the arginine

residue found at position 1 in the native rat-derived Atrial Natriuretic Peptide sequence disclosed in Atlas, S., et al., Nature (1984) 309:717-719.

## I. Chemical Synthesis of Atrial Natriuretic Peptide Analog Compounds

Compounds of the present invention were synthesized by solid-phase techniques performed manually or, alternatively, on an Applied BioSystems 430A Peptide Synthesizer (Foster City, California) or a Biosearch Sam II automated peptide synthesizer (Biosearch, San Rafael, California) using t-Boc amino acids in accordance with the instructions of the manufacturer.

### Procedure A

### Preparation of Boc-$AA_1$...$AA_{n-1}$-$AA_n$-Resin Hydroxymethyl Polystyrene Ester

One gram of selected Boc-$AA_n$-O-Polystyrene-Resin (0.2-0.6 mmol/g resin) (obtainable from, e.g., Peninsula Labs, Inc.) is treated according to schedule A for incorporation of the Boc-$AA_{n-1}$-OH.

Schedule A

1) Wash 3x with dichloromethane ($CH_2Cl_2$);
2) Treat for 1 min. with TFA:$CH_2Cl_2$:ethane dithiol (EDT) (45:50:5 by volume);
3) Treat for 20 min. with TFA:$CH_2Cl_2$:EDT (45:50-5) by volume;
4) Wash 3x with $CH_2Cl_2$;
5) Treat 2x for 1 min. 10% (V/V) Diisopropylethylamine (DIPEA) in $CH_2Cl_2$;
6) Wash 2x with $CH_2Cl_2$;
7) Wash 2x with methanol (MeOH);
8) Repeat (5-7) once;
9) Wash 3x with $CH_2Cl_2$;
10) Add 1-6 equivalents of preformed symmetrical anhydride of the suitably protected Boc-amino acid dissolved in $CH_2Cl_2$ or dimethyl formamide (DMF)/$CH_2Cl_2$ (50:5) volume), (Boc-Asn-OH, Boc-Gln-OH and Boc-Arg(TOS)-OH were coupled as active esters using N-hydroxybenzotriazole);
11) Wash 2x with $CH_2Cl_2$;
12) Wash 2x with 10% DIPEA;
13) Wash 2x with $CH_2Cl_2$;
14) Wash 2x with MeOH;
15) Wash 2x with $CH_2Cl_2$;
16) Repeat steps (11-15) once;
17) Test by ninhydrin reaction according to Kaiser et al., Anal. Biochem. 34:595 (1970). If the coupling reaction was incomplete, repeat steps (10-16) or, alternatively, cap synthesis using N-acetyl imidazole (0.30 M in DMF) or an excess of acetic anhydride in $CH_2Cl_2$.

### Procedure B

### Preparation of Boc-$AA_n$-p-Methylbenzhydrylamine resin

The selected Boc-$AA_n$-OH is attached to a p-Methylbenzhydrylamine (pMBHA) resin via N,N′-dicyclohexylcarbodiimide, as described below.

Schedule B

1) Was the pMBHA HCl resin;
2) Wash the resin 2x with 10% (V/V) DIPEA in $CH_2Cl_2$;
3) Wash 2x with $CH_2Cl_2$;
4) Wash 2x with MeOH;
5) Wash 2x with $CH_2Cl_2$;
6) Add 1-6 equivalents of preformed symmetrical anhydride of the suitably protected Boc-amino acid dissolved in $CH_2Cl_2$, with reaction time of 0.5 - 24 hrs.

Unreacted amino groups are acetylated with 0.30M N-acetylimidazole:DMF, or acetic anhydride:$CH_2Cl_2$. The following examples demonstrate the chemical synthesis of representative analog ANP compounds (identified as AP#) which illustrate certain aspects of the present invention.

The compounds of the invention are prepared as illustrated below, and incorporated here from WO87/02674 for convenience.

Example 306

* AP306 (2-Naphthylacetyl)-G-G-R-I-D-R-I-G-A-$NH_2$

One gm of Boc-Ala-pMBHA resin (0.4 meq/gm), obtained using schedule B, was subjected to procedure A with the required sequence of amino acids and Amino-terminal substituent group (introduced in order to Boc-Gly-OH, Boc-Ile-OH 1/2$H_2O$, Boc-Arg(Tos)-OH, Boc-Asp(OBzl)-OH, Boc-Ile-OH 1/2$H_2O$, Boc-Arg(Tos)-OH, Boc-Gly-OH, Boc-Gly-OH, 2-Naphthalyacetic acid). The protected peptidyl resin was washed 3 times with $CH_2Cl_2$ and 3 times with MeOH and dried in vacuo.

The peptidyl resin was then suspended in anhydrous HF containing 10% anisole, 2% ethyl methyl sulfide for 30 min. at -10°C and for 30 min. at 0°C. The HF was removed by evaporation under vacuum and the peptide/resin mixture was suspended in ethyl ester. After transfer to a fritted funnel, the peptide/resin mixture was washed twice with ethyl ether, once with chloroform, once with ethyl ether, once with chloroform and once again with ethyl ether. The peptide was then extracted from the mixture with 2.0 M acetic acid, diluted with $H_2O$ and lyophilized.

Purification of the peptide was achieved by ion exchange chromatography on CM-Sepharose® (Pharmacia) using an elution gradient generated by addition of 100 mM $NH_4OAc$, pH 6.5, to a solution of 10 mM $NH_4OAc$, pH 4.5. Fractions were monitored at 254 nm and analyzed by reversed phase HPLC. Fractions having a minimum 97% purity were pooled and lyophilized from $H_2O$ several times to yield the purified AP306 acetate salt.

Example 307

* AP307 (2-Naphthoxyacetyl)-$NH(CH_2)_4CO$-R-I-D-R-I-$NH_2$

One gm of Boc-Ile-pMBHA resin (0.4 meq/gm), obtained using schedule B, was subjected to procedure A with the required sequence of amino acids and Amino-terminal substituent group (introduced in order as Boc-Arg(Tos)-OH, Boc-Asp(OBzl)-OH, Boc-Ile-OH 1/2$H_2O$, Boc-Arg(Tos)-OH, Boc-$NH(CH_2)_4COOH$, 2-Naphthoxyacetic acid). The protected peptidyl resin was washed three times with $CH_2Cl_2$ and three times with MeOH and dried in vacuo.

The peptidyl resin was then suspended in anhydrous HF containing 10% anisole, 2% ethyl methyl sulfide for 30 min. at -10°C and for 30 min. at 0°C. The HF was removed by evaporation under vacuum

and the peptide/resin mixture was suspended in ethyl ether. After transfer to a fritted funnel, the peptide/resin mixture was washed twice with ethyl ether, once with chloroform, once with ethyl ether, once with chloroform and once again with ethyl ether. The peptide was then extracted from the mixture with 2.0 M acetic acid, diluted with $H_2O$ and lyophilized.

Purification of the peptide was achieved by ion exchange chromatography on CM-Sepharose® (Pharmacia) using an elution gradient generated by addition of 100 mM $NH_4OAc$, pH 6.5, to a solution of 10 mM $NH_4OAc$, pH 4.5. Fractions were monitored at 254 nm and analyzed by reversed-phase HPLC. Fractions having a minimum 97% purity were pooled and lyophilized from $H_2O$ several times to yield the purified AP307 acetate salt.

Following the procedures outlined in Examples 306 and 307 (to produce analog peptides AP306 and AP307) with appropriate modification, the ANP analogs set forth in figures 2 and 3 were synthesized. In the figure, the following abbreviations as set forth above are used, including the following:

AA = Adamantylacetyl
BPA = Biphenylacetyl
CHA = Cyclohexylacetyl
DBA = Dibenzylacetyl
DPP = Diphenylpropionyl
IB = Indolebutyryl
IP = Indoleproprionyl
NA = Naphthylacetyl
NL = Naphthyl
NM = Naphthylmethyl
NO = Naphthoxy
NOA = Naphthoxyacetyl
NTA = Naphthylthioacetyl
NYL = Naphthoyl
POP = Phenoxypropionyl
TPP = Triphenylpropionyl
MeONAP = Methoxynaphthylpropionyl

In each of the starred examples, amino acid analysis demonstrated that the appropriate amino acid sequence of the peptide was obtained.

## II. Biological Testing: Receptor Binding Assays

Biological activity data for selected analog Atrial Natriuretic Peptides (ANPs) of the invention which were synthesized as disclosed above are presented below as results of receptor binding assays. Correlation with isolated tissue and whole mammal bioassays was set forth in WO87/02674.

Without intending to be bound by any theory, it is believed that the activity of the ANP analog compounds of the invention is due to their affinity for receptors in the kidney and other sites which are responsible for influencing the clearance of the endogenous ANPs. The following in vitro biological data shown that the analog compounds of the invention compete with an iodinated native ANP molecule for binding to receptors from cultured bovine aortic smooth muscle (BASM) cells, and bovine endothelial (BAE) cells. This competition is, evidently, diagnostic for the binding to the relevant clearance receptors. This correlation is confirmed by data in WO87/02674 as set forth above. In addition, the analogs of the invention show reduced cyclic GMP activity, an activity which is a hallmark of the direct biological function of ANP.

It is also postulated by the inventors some, if not many, of the peptides and peptide analogs disclosed herein will have oral activity as well.

Specific ANP receptor sites have been identified on target tissues, such as kidney, adrenal, blood vessels, and cultured cells. Napier, M.A., et al., Proc Nat Acad Sci USA (1984) 81:5946-5940 ; DeLean, A., et al., Endocrinology (1984) 115:1636-1638 : Schenk, D.B., et al., Biochem Biophys Res Comm (1985) 127:433-442. Since the binding of ANP or ANP analogs to these specific receptor sites is presumptively a prerequisite of biological activity, binding of ANP analogs to these receptors is considered predictive of biological activity.

An assay has been developed, generally in accordance with the disclosure of Schenk, supra, and Scarborough, R.M., et al., J. Biol Chem (1986) 261:12960-12964, which evaluates the ability of ANP analogs to compete with labeled native ANP for binding to cultured BASM and BAE cells. This native ANP, having the amino acid sequence:

126 150
R-S-S-C-F-G-G-R-I-D-R-I-G-A-Q-S-G-L-G-C-N-S-F-R-Y

was iodinated on the carboxy-terminal Y residue and is identified as ($^{125}$I)-rANP(126-150). Analogous "competitive displacement" receptor binding assays are considered commonplace in the art for examining specific ligand-receptor interactions.

In this assay, 0.5 nM ($^{125}$I)-rANP(126-150) is incubated in each individual sample of BASM cells in the presence of varying amounts of unlabeled rANP(126-150) or the test compound. The concentration of unlabeled peptide at which 50% of maximal ($^{125}$I)-rANP(126-150) binding is displaced is called Ki(app) and reflects receptor-binding affinity. Therefore, hypothetical peptide A, with a Ki(app) = 100nM, displays substantially weaker interaction with a receptor than hypothetical peptide B with a Ki(app) = 10nM. Assuming these ANP analogs act at one or more ANP receptor sites, then increased receptor affinity should reflect increased biological potency.

Tables 1-5 present data which compare the concentrations at which analog compounds of the invention displace ($^{125}$I)-rANP(126-150) binding from specific receptor sites on BASM or BAE cells.

Table 1

| | | |
|---|---|---|
| AP524 | (2-NA)-G-G-R-I-D-R-I-G-NH-(ethyl) | 2.76 |
| AP525 | (2-NA)-G-G-R-I-D-R-I-G-(Aib)-$NH_2$ | 22.75 |
| AP526 | (2-NA)-G-G-R-I-D-R-I-NH-$(CH_2)_4$-$CONH_2$ | 50.5 |
| AP528 | 2(2'-(6'-MeONAP))-G-G-R-I-D-R-I-G-A-$NH_2$ | 22.5 |
| AP530 | (2-NA)-NH$(CH_2)_4$CO-R-I-D-R-I-G-NH-(ethyl) | 50.5 |
| AP531 | (3-IB)-G-R-I-D-R-I-G-A-$NH_2$) | 9.34 |
| AP532 | (3-IB)-beta-Ala-R-I-D-R-I-G-A-$NH_2$) | 11.95 |
| AP533 | (bis-(1'-NM)acetyl)-G-G-R-I-D-R-I-G-A-$NH_2$) | 52.0 |
| AP534 | (DBA)-G-G-R-I-D-R-I-G-A-$NH_2$) | 8.81 |
| AP576 | (2-NOA)-NH$(CH_2)_3$CO-R-I-D-R-I-G-A-$NH_2$ | 16.0 |
| AP597 | (3-IB)-G-R-I-D-R-I-G-A-$NH_2$ | 9.3 |

All of the foregoing compounds contain the preferred R(I/M)DRI core per se.

| | | |
|---|---|---|
| AP518 | (2-NA)-G-G-R-I-E-R-I-$NH_2$ | 38 |
| AP522 | (2-NA)-G-G-Q-I-D-R-I-$NH_2$ | 23.0 |

| | | |
|---|---|---|
| AP523 | (2-NA)-G-G-K-I-D-R-I-$NH_2$ | 36.7 |
| AP527 | (2-NA)-G-G-R-Cha-D-R-I-$NH_2$ | 22.5 |
| AP529 | (2-NA)-G-G-R-I-D-R-N-MeIle-G-NH-(ethyl) | 87.3 |
| AP535 | (2-NA)-G-G-R-I-D-R-V$^{\dagger}$-$NH_2$) | 14.0 |
| AP537 | (2-NA)-G-G-R-I-D-R-F$^{\dagger}$-$NH_2$) | >160 |
| AP590 | (2-NA)-G-G-R-tBuA-D-R-I-$NH_2$ | 12.0 |
| AP591 | (2-NA)-G-G-R-tBuG-D-R-I-$NH_2$ | 14.0 |
| AP592 | (2-NA)-G-G-R-I-D-Orn-I-$NH_2$ | 25.0 |
| AP593 | (2-NA)-G-G-R-I-D-Cit-I-$NH_2$ | 55.0 |
| AP594 | (2-NA)-G-G-R-I-D-R-tBuA-$NH_2$ | 15.0 |
| AP595 | (2-NA)-G-G-R-I-D-R-Phg-$NH_2$ | 100.0 |
| AP596 | (2-NA)-G-G-R-I-D-R-NMeIle-NH(ethyl) | 87.3 |
| AP599 | (2-NA)-G-G-R-Cha-D-R-I-$NH_2$ | 22.5 |
| AP333 | (2-NA)-G-G-R-I-D-R-V-$NH_2$ | 106.4 |
| AP536 | (2-NA)-G-G-R-I-D-R-L$^{\dagger}$-$NH_2$) | 106.4 |
| AP512 | (2-NA)-G-G-L-I-D-R-I-$NH_2$ | >160 |
| AP514 | (2-NA)-G-G-Nle-I-D-R-I-$NH_2$ | 267.0 |
| AP515 | (2-NA)-G-G-R-MSO-D-R-I-$NH_2$ | >400 |

| | | |
|---|---|---|
| AP516 | (2-NA)-G-G-R-I-D-S-I-$NH_2$ | 399 |
| AP519 | (2-NA)-G-G-R-I-D-R-P-$NH_2$ | >400 |
| AP521 | (2-NA)-G-G-R-F-D-R-I-$NH_2$ | 400 |
| AP575 | (2-NA)-G-G-R-Phg-D-R-I-$NH_2$ | 194.0 |
| AP789 | (2-NOA)-NH-$(CH_2)_7$-CO-R-I-D-R-I-$NH_2$ | 380 |

In the compounds above, the $Z_2$ spacer is $(AA)_a$ or $HN(CH_2)_mCO$-. In these cases, $Z_3$ is $NH_2$, $NHR'$, or a very short peptide of 1-3 amino acid residues, or an amide or alkyl amide thereof.

Compounds having relatively low activity as judged by Ki are placed at the end of the table. AP512, AP514, AP515, AP516, AP519, AP521 and AP575 have very low, if any, activity. All except AP519 fall outside the invention compounds, as lacking the required pentapeptide core; AP519 is not a preferred embodiment.

Table 2 shows the activity of compounds analogous to those of Table 1, but with spacers of the formula $-(Q)_m-B-(Q)_m-(CO)_x-$.

Table 2

| Peptide | Sequence | Ki(app)(nM) |
|---|---|---|
| AP538 | (2-NA)- 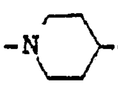 -CO-R-I-D-R-I-$NH_2$ | 9.9 |
| AP539 | (2-NOA)- 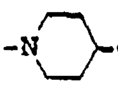 -CO-R-I-D-R-I-$NH_2$ | 9.1 |
| AP560 | (2-NA)-NH- 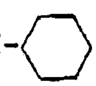 -CO-R-I-D-R-I-$NH_2$ | 68.8 |
| AP562 | (2-NYL)- 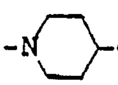 -CO-R-I-D-R-I-$NH_2$ | 22.4 |

| | | |
|---|---|---|
| AP564 | (2-NA)-NH-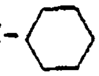-$CH_2$CO-R-I-D-R-I-$NH_2$ | 10.1 |
| AP568 | (2-NA)-NH$CH_2$-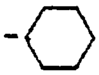-CO-R-I-D-R-I-$NH_2$ | 12.5 |
| AP574 | (2-NYL)-$CH_2$-N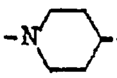-CO-R-I-D-R-I-$NH_2$ | 26.5 |
| AP704 | (2-NA)-N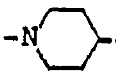-$CH_2$-R-I-D-R-I-$NH_2$ | 17.0 |
| AP790 | (2-NYL)-NH-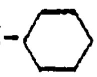-$CH_2$-CO-R-I-D-R-I-$NH_2$ | 5.2 |
| AP791 | (2-NOA)-NH-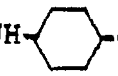-CO-R-I-D-R-I-$NH_2$ | 31.1 |
| AP792 | (2-NOA)-NH-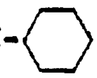-$CH_2$-CO-R-I-D-R-I-$NH_2$ | 6.3 |
| AP793 | (2-NOA)-N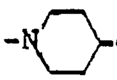-CO-R-I-D-R-I-$NH_2$ | 76.8 |
| AP794 | (2-NYL)-$CH_2$NH-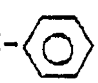-CO-R-I-D-R-I-$NH_2$ | 13.1 |
| AP795 | (2-NYL)-NH$CH_2$-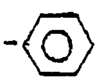-CO-R-I-D-R-I-$NH_2$ | 11.9 |
| AP796 | (2-NL)-$CH_2$$CH_2$NH-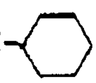-$CH_2$-CO-R-I-D-R-I-$NH_2$ | 27.3 |
| AP816 | (2-NOA)-NH-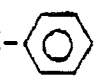-$CH_2$($CH_2$NH)-R-I-D-R-I-$NH_2$ | 17.4 |
| AP817 | (2-ClPA)-NH-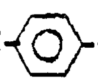-$CH_2$CO-R-I-D-R-I-$NH_2$ | 21.1 |
| AP818 | (2-BrPA)-NH-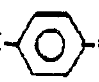-$CH_2$CO-R-I-D-R-I-$NH_2$ | 20.5 |
| AP819 | (2-BrAF)-NH-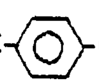-$CH_2$CO-R-I-D-R-I-$NH_2$ | 3.8 |

Table 3 shows compounds similar to those of Table 2 except that the core R(I/M)DRI sequence contains at least one substitution, preferably K or (Acetyl) Lys rather than R, or L rather than (I/M).

Table 3

| Peptide | Sequence | Ki(app)(nM) |
|---|---|---|
| AP798 | (2-NOA)-NH-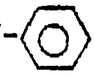-$CH_2$-CO-K-I-D-R-I-$NH_2$ | 3.95 |
| AP799 | (2-NA)-NH-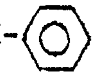-$CH_2$CO-R-I-D-K-I-$NH_2$ | 3.6 |
| AP800 | (2-NA)-NH-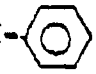-$CH_2$-CO-K-I-D-R-I-$NH_2$ | 2.90 |
| AP801 | (2-NOA)-NH-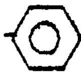-$CH_2$-CO-(Acetyl)Lys-I-D-R-I-$NH_2$ | 3.95 |
| AP802 | (2-NYL)-NH-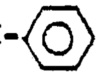-$CH_2$CO-K-I-D-R-I-$NH_2$ | 8.7 |
| AP809 | (2-NYL)-NH-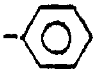-$CH_2$CO-K($CH_2$NH)-I-D-R-I-$NH_2$ | 14.7 |
| AP814 | (2-NYL)-NH-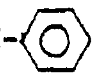-$CH_2$CO-K($CH_2$NH)-I-D-R-NH-(S)-2-methylbutyl | 33.0 |
| AP815 | (2-NOA)-NH-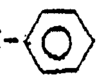-$CH_2$CO-Orn-I-D-R-I-$NH_2$ | 10.0 |

Table 4 shows the results for compounds which have non-peptide bond linkages in the core sequence, but contain otherwise unmodified R(I/M)DRI; or have only one other modification therein.

Table 4

| Peptide | Sequence | Ki(app)(nM) |
|---|---|---|
| AP701 | (2-NA)-NH-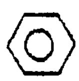-$CH_2$CO-R-I($CH_2$NH)D-R-I-$NH_2$ | 60.3 |
| AP806 | (2-NA)-NH-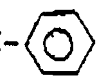-$CH_2$CO-K($CH_2$NH)I-D-R-I-$NH_2$ | 14.0 |
| AP807 | (2-NA)-NH-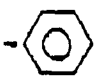-$CH_2$-CO-R-I-D-K($CH_2$NH)I-$NH_2$ | 112 |
| AP808 | (2-NOA)-NH-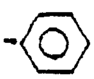-$CH_2$CO-K($CH_2$NH)I-D-R-I-$NH_2$ | 98.9 |

Table 5 shows the activity of embodiments wherein $A_{12}$ is a covalent bond; other modifications may also apply.

Table 5

| Peptide | Sequence | Ki(app)(nM) |
|---|---|---|
| AP509 | (2-NOA)-G-G-R-I-D-R-NH-$CH_2$-$CH(CH_3)_2$ | 120.0 |
| AP510 | (2-NA)-G-G-R-I-D-R-NH-$CH_2$-$CH(CH_3)_2$ | 116.0 |
| AP511 | (2-NA)-G-G-R-I-D-R-NH-$CH_2$-$CH(CH_3)CH_2CH_3$ | 84.7 |
| AP557 | (2-NA)-N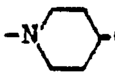-CO-R-I-D-R-NH-$CH_2$-$CH(CH_3)CH_2CH_3$ | 41.7 |
| AP816 | (2-NOA)-N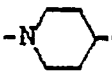-CO-R-I-D-R-NH-(S)-2-methylbutyl | 14.1 |
| AP811 | (2-NYL)-NH-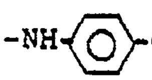-$CH_2CO$-R-I-D-R-NH-(S)-2-methylbutyl | 2.52 |
| AP812 | (2-NOA)-N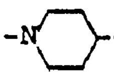-$CH_2NH$-R-I-D-R-NH-(S)-2-methylbutyl | 32.0 |
| AP813 | (2-NOA)-NH-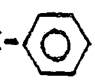-$CH_2CO$-R-I-D-R-NH-(S)-2-methylbutyl | 56.0 |

These data indicate the Ile at $A_{12}$ is not essential for activity of $Z_3$ is NHR′, wherein R′ is alkyl, in the foregoing examples, of 4-5C.

In order to show that the receptor binding assay is specific to ANP, data which compares ANP-receptor interactions of rANP(126-150) with the unrelated peptide hormones angiotensin II, glucagon, parathyroid hormone and gamma-MSH is shown:

| Peptide | Ki(app) |
|---|---|
| rANP(126-150) | 7.50 |
| angiotensin II | >500 |
| glucagon | >500 |
| parathyroid hormone | >500 |
| gamma-MSH | >500 |

As shown above, only rANP(126-150) displays detectable ANP-receptor affinity. This attests to the relevant ANP-specificity of this receptor.

The data in the foregoing tables shown that a large representative sample of the compounds of the invention demonstrate affinity in the specific receptor-binding assay described.

Although the foregoing invention has been illustrated above for purposes of aiding understanding, modifications of the invention may be practiced while remaining within the spirit and scope of the appended claims.

## Claims

1. A linear peptide compound, having natriuretic, diuretic and/or vasodilator activity in mammals, which has the formula:

$Z_1Z_2$-$AA_8$-$AA_9$-$AA_{10}$-$AA_{11}$-$AA_{12}$-$Z_3$ (1)

wherein:

each of $AA_8$ and $AA_{11}$ is, independently, a basic/noncyclic; neutral/nonpolar/small; or

neutral/polar/large/nonaromatic amino acid residue; and $AA_8$ can also be a neutral nonpolar/large/nonaromatic amino acid residue;

$AA_9$ is a neutral/nonpolar/large/nonaromatic amino acid residue in the D or L configuration;

$AA_{10}$ is an acidic amino acid residue;

$AA_{12}$ is a neutral/nonpolar/large/nonaromatic amino acid residue in the D or L configuration or a covalent bond; and

wherein $Z_1$ is a peptide of from 1 to 125 amino acids having as its carboxy-terminal residue a hydrophobic amino acid residue, or the desNH$_2$ form thereof, or is a hydrophobic aliphatic, aromatic, or mixed aliphatic/aromatic organic group of from 6 to 20 carbon atoms;

$Z_2$ is a spacer group capable of providing a spaced dimension of 4.5-15 angstroms between $AA_8$ and the hydrophobic moiety of $Z_1$;

$Z_3$ is (OH), $NH_2$, $NHR'$ or $NR'R''$ wherein $R'$ or $R''$ are each independently straight or branched chain alkyl of 1-10 carbon atoms wherein 1 or 12 carbons may be replaced by O, N, or S; or is a peptide of 1-20 amino acid residues, or an amide or alkyl amide thereof, with the proviso that when $AA_{12}$ is a covalent bond, $Z_3$ cannot be (OH), $NH_2$ or a peptide; and

wherein one or more of the amide linkages between adjacent amino acid residues is replaced by a linkage selected from the group consisting of $-CH_2NH-$, $-CH_2S-$, $-CH_2CH_2-$, $-CH=CH-$, $-COCH_2-$, $-CH(OH)CH_2-$ and $-CH_2SO-$.

2. A linear peptide compound, having natriuretic, diuretic and/or vasodilator acitivity in mammals, which has the formula:

$Z_1Z_2\text{-}AA_8\text{-}AA_9\text{-}AA_{10}\text{-}AA_{11}\text{-}AA_{12}\text{-}Z_3$ (1)

wherein:

each of $AA_8$ and $AA_{11}$ is, independently, a basic/noncyclic; neutral/nonpolar/small; or neutral/polar/large/nonaromatic amino acid residue; and $AA_8$ can also be a neutral nonpolar/large/nonaromatic amino acid residue;

$AA_9$ is a neutral/nonpolar/large/nonaromatic amino acid residue in the D or L configuration;

$AA_{10}$ is an acidic amino acid residue;

$AA_{12}$ is a covalent bond; and

wherein $Z_1$ is a peptide of from 1 to 125 amino acids having as its carboxy-terminal residue a hydrophobic amino acid residue, or the desNH$_2$ form thereof, or is a hydrophobic aliphatic, aromatic, or mixed aliphatic/aromatic organic group of from 6 to 20 carbon atoms;

$Z_2$ is a spacer group capable of providing a spaced dimension of 4.5-15 angstroms between $AA_8$ and the hydrophobic moiety of $Z_1$;

$Z_3$ is $NHR'$ or $NR'R''$ wherein $R'$ or $R''$ are each independently straight or branched chain alkyl of 1-10 carbon atoms wherein 1 or 2 carbons may be replaced by O, N, or S; and

wherein one or more of the amide linkages between adjacent amino acid residues may optionally be replaced by a linkage selected from the group consisting of $-CH_2CH-$, $-CH_2S-$, $-CH_2CH_2-$, $-CH=CH-$, $-COCH_2-$, $-CH(OH)CH_2-$ and $-CH_2SO-$.

3. A linear peptide compound, having natriuretic, diuretic and/or vasodilator activity in mammals, which has the formula:

$Z_1Z_2\text{-}AA_9\text{-}AA_{10}\text{-}AA_{11}\text{-}AA_{12}\text{-}Z_3$ (1)

wherein:

each of $AA_8$ and $AA_{11}$ is, independently, a basic/noncyclic; neutral/nonpolar/small; or neutral/polar/large/nonaromatic amino acid residue; and $AA_8$ can also be a neutral nonpolar/large/nonaromatic amino acid residue;

$AA_9$ is a neutral/nonpolar/large/nonaromatic amino acid residue in the D or L configuration;

$AA_{10}$ is an acidic amino acid residue;

$AA_{12}$ is a neutral/nonpolar/large/nonaromatic amino acid residue in the D or L configuration or a covalent bond; and

wherein $Z_1$ is a peptide of from 1 to 125 amino acids having as its carboxy-terminal residue a hydrophobic amino acid residue, or the desNH$_2$ form thereof, or is a hydrophobic aliphatic, aromatic, or mixed aliphatic/aromatic organic group of from 6 to 20 carbon atoms;

$Z_2$ is a spacer group capable of providing a spaced dimension of 4.5-15 angstroms between $AA_8$ and the hydrophobic moiety of $Z_1$, selected from the group consisting of:

$-(P)_n-(CO)_x-$wherein x is 0 or 1, n is 1-6, and P is $CH_2$, wherein 1-2 of said $-CH_2-$ groups can be replaced by NH, provided N-N does not occur, and

$-(Q)_m-B-(Q)_m-(CO)_x-$wherein x is 0 or 1, each m is independently 0-3 but the sum of both m is 5 or less; Q is $CH_2$ or NH, with the proviso that -N-N- does not occur, and B is a saturated or unsaturated five- or six-membered ring optionally containing an N heteroatom, wherein B can be joined to Q either 1, 4 or 1, 3, with the proviso that $Z_2$ cannot be -NH($CH_2$)3-6CO-;
$Z_3$ is (OH), $NH_2$, NHR′ or NR′R″ wherein R′ or R″ are each independently straight or branched chain alkyl of 1-10 carbon atoms wherein 1 or 2 carbons may be replaced by O, N, or S; or is a peptide of 1-20 amino acid residues, or an amide or alkyl amide thereof, with the proviso that when $AA_{12}$ is a covalent bond, $Z_3$ cannot be (OH), $NH_2$ or a peptide; and
wherein one or more of the amide linkages between adjacent amino acid residues may optionally be replaced by a linkage selected from the group consisting of $-CH_2NH-$, $-CH_2S-$, $-CH_2CH_2-$, -CH=CH-, $-COCH_2-$, $-CH(OH)CH_2-$ and $-CH_2SO-$.

4. A linear peptide compound, having natriuretic, diuretic and/or vasodilator activity in mammals, which has the formula:
$Z_1Z_2$-$AA_8$-$AA_9$-$AA_{10}$-$AA_{11}$-$AA_{12}$-$Z_3$ (1)
wherein:
each of $AA_8$ and $AA_{11}$ is, independently, a basic/noncyclic; neutral/nonpolar/small; or neutral/polar/large/nonaromatic amino acid residue; and $AA_8$ can also be a neutral nonpolar/large/nonaromatic amino acid residue;
$AA_9$ is a neutral/nonpolar/large/nonaromatic amino acid residue in the D or L configuration;
$AA_{10}$ is an acidic amino acid residue;
$AA_{12}$ is a neutral/nonpolar/large/nonaromatic amino acid residue in the D or L configuration or a covalent bond, and
wherein $Z_1$ is of the formula $R'_1CO$, $R'_1COCH_2$, $R'_1O$- or $R'_1$- wherein $R'_1$ contains at least one halo group alpha to a cabonyl moiety;
$Z_2$ is a spacer group capable of providing a spaced dimension of 4.5-15 angstroms between $AA_8$ and the hydrophobic moiety of $Z_1$;
$Z_3$ is (OH), $NH_2$, NHR′ or NR′R″ wherein R′ or R″ are each independently straight or branched chain alkyl of 1-10 carbon atoms wherein 1 or 2 carbons may be replaced by O, N, or S; or is a peptide of 1-20 amino acid residues, or an amide or alkyl amide thereof, with the proviso that when $AA_{12}$ is a covalent bond, $Z_3$ cannot be (OH), $NH_2$ or a peptide; and
wherein one or more of the amide linkages between adjacent amino acid residues may optionally be replaced by a linkage selected from the group consisting of $-CH_2NH-$, $-CH_2S-$, $-CH_2CH_2-$, -CH=CH-, $-COCH_2-$, $-CH(OH)CH_2-$ and $-CH_2SO-$.

5. The compound of claims 1-3 wherein $Z_1$ is selected from the group consisting of fluorenylmethyloxycarbonyl, benzyloxycarbonyl, 2-(2′-(6′-methoxy naphthyl)) propionyl, diphenylpropionyl, biphenylacetyl, triphenylpropionyl, cyclohexylacetyl, 3-indolepropionyl, 4-indolebutyryl, 1-adamantylacetyl, 1-naphthylacetyl, 2-naphthylacetyl, 1-naphthoxyacetyl, 2-naphthoxyacetyl, dibenzylacetyl, bis (1′-napthylmethyl) acetyl, 2-naphthyl thioacetyl, 3-phenoxypropionyl, 2-naphthoyl, 2-naphthoxy, 2-naphthyl, phenylalanyl and des-$NH_2$ phenylalanyl.

6. The compound of claim 4 wherein $Z_1$ is selected from the group consisting of 2-halo phenylacetyl and 2-halo acetyl phenylalanyl

7. The compound of claims 1-4 wherein $Z_2$ is 4-APA.

8. The compound of claims 1, 3, or 4 wherein $AA_8$-$AA_9$-$AA_{10}$-$AA_{11}$-$AA_{12}$ is R(I/M)DRI and at most one residue therein is replaced by substituting
K, (Acetyl) Lys, A, Q, N, L or NMeIle for R as $AA_8$
V, V†, L, L†, I†, M†, t-BuA, t-BuG or Cha for I or M as $AA_9$;
E for D as $A_{10}$;
K, (Acetyl) Lys, A, Q, N, Orn or Cit for R as $A_{11}$; and
M, M†, V, V†, L, L†, I†, P, N-MeIle, t-Bua or a covalent bond for I as $AA_{12}$.

9. The compound of claim 8 wherein $AA_8$-$AA_9$-$AA_{10}$-$AA_{11}$-$AA_{12}$ is selected from the group consisting of:

| | | |
|---|---|---|
| A(I/M)DRI<br>K(I/M)DRI | RM†DRI<br>RLDRI | R(I/M)DRL<br>R(I/M)DRM |
| (Acetyl) Lys (I/M)DRI | | |
| Q(I/M)DRI<br>RVDRI<br>RI†DRI | R(I/M)ERI<br>R(I/M)DKI<br>R(I/M)DQI | R(I/M)DRM†<br>R(I/M)DRI†<br>R(I/M)DRV |

10. A composition useful as a natriuretic, diuretic and/or vasodilator comprising a therapeutically effective amount of the compound of claims 1-8 together with a pharmaceutically acceptable carrier.

11. A process for production of a peptide compound having natriuretic, diuretic and/or vasodilator activity in mammals, said peptide compound having the formula of the compound of claims 1-8, or the pharmacologically acceptable salts thereof, which process comprises the following steps:

a. preparing a protected peptide bonded to a solid resin carrier in a reaction mixture, wherein the peptide has an amino acid sequence as recited above;

b. removing the solid resin carrier from the peptide and deprotecting the peptide;

c. optionally modifying the peptide to add any desired organic substituent groups as recited above; and

d. isolating the peptide from any reaction mixture, and optionally, convering the polypeptide into an acid addition salt thereof.

FIGURE 2



* Indicates amino acid analysis has been performed.

| | | |
|---|---|---|
| * | AP100 | A-F-G-G-R-I-D-R-I-G-A-A-$NH_2$ |
| * | AP101 | A-F-G-G-R-I-D-R-I-G-A-$NH_2$ |
| * | AP102 | A-F-G-G-R-I-D-R-I-G-$NH_2$ |
| * | AP103 | A-F-G-G-R-I-D-R-I-$NH_2$ |
| * | AP104 | F-G-G-R-I-D-R-I-G-A-A-$NH_2$ |
| | AP118 | A-F-G-G-R-M-D-R-I-G-$NH_2$ |
| * | AP119 | F-G-G-R-I-D-R-I-$NH_2$ |
| * | AP122 | F-G-G-R-I-D-R-I-G-$NH_2$ |
| * | AP126 | F-G-G-R-I-D-R-I-G-A-$NH_2$ |
| * | AP130 | (des$NH_2$-F)-G-G-R-I-D-R-I-G-A-$NH_2$ |
| | AP131 | (des$NH_2$-F)-G-G-R-I-D-R-I-G-$NH_2$ |
| * | AP132 | (des$NH_2$-F)-G-G-R-I-D-R-I-$NH_2$ |
| * | AP133 | A-F-A$^{\dagger}$-G-R-I-D-R-I-G-A-$NH_2$ |
| | AP134 | A-F-A$^{\dagger}$-G-R-I-D-R-I-G-$NH_2$ |
| | AP135 | A-F-A$^{\dagger}$-G-R-I-D-R-I-$NH_2$ |
| | AP136 | F-A$^{\dagger}$-G-R-I-D-R-I-G-A-$NH_2$ |

AP137 $F-A^{\dagger}-G-R-I-D-R-I-G-NH_2$

AP138 $F-A^{\dagger}-G-R-I-D-R-I-NH_2$

AP139 $(desNH_2-F)-A^{\dagger}-G-R-I-D-R-I-G-A-NH_2$

AP140 $(desNH_2-F)-A^{\dagger}-G-R-I-D-R-I-G-NH_2$

AP141 $(desNH_2-F)-A^{\dagger}-G-R-I-D-R-I-NH_2$

AP142 $A-F-S^{\dagger}-G-R-I-D-R-I-G-A-NH_2$

AP143 $A-F-S^{\dagger}-G-R-I-D-R-I-G-NH_2$

AP144 $A-F-S^{\dagger}-G-R-I-D-R-I-NH_2$

AP145 $F-S_{\dagger}-G-R-I-D-R-I-G-A-NH_2$

AP146 $F-S_{\dagger}-G-R-I-D-R-I-G-NH_2$

AP147 $F-S_{\dagger}-G-R-I-D-R-I-NH_2$

AP148 $(desNH_2-F)-S^{\dagger}-G-R-I-D-R-I-G-A-NH_2$

AP149 $(desNH_2-F)-S_{\dagger}-G-R-I-D-R-I-G-NH_2$

AP150 $(desNH_2-F)-S^{\dagger}-G-R-I-D-R-I-NH_2$

* AP151 $A-F^{\dagger}-G-G-R-I-D-R-I-G-A-NH_2$

AP152 $A-F^{\dagger}-G-G-R-I-D-R-I-G-NH_2$

AP153 $A-F^{\dagger}-G-G-R-I-D-R-I-NH_2$

AP154 $F^{\dagger}$-G-G-R-I-D-R-I-G-A-$NH_2$

AP155 $F_{\dagger}$-G-G-R-I-D-R-I-G-$NH_2$

AP156 $F^{\dagger}$-G-G-R-I-D-R-I-$NH_2$

AP157 A-F-G-$A^{\dagger}$-R-I-D-R-I-G-A-$NH_2$

AP158 A-F-G-$A^{\dagger}$-R-I-D-R-I-G-$NH_2$

AP159 A-F-G-$A_{\dagger}$-R-I-D-R-I-$NH_2$

AP160 F-G-$A^{\dagger}$-R-I-D-R-I-G-A-$NH_2$

AP161 F-G-$A^{\dagger}$-R-I-D-R-I-G-$NH_2$

AP162 F-G-$A_{\dagger}$-R-I-D-R-I-$NH_2$

AP163 (des$NH_2$-F)-G-$A^{\dagger}$-R-I-D-R-I-G-A-$NH_2$

AP164 (des$NH_2$-F)-G-$A^{\dagger}$-R-I-D-R-I-G-$NH_2$

AP165 (des$NH_2$-F)-G-$A^{\dagger}$-R-I-D-R-I-$NH_2$

AP166 A-F-G-G-R-I-D-R-I-G-$A_{\dagger}$-$NH_2$

AP167 F-G-G-R-I-D-R-I-G-$A^{\dagger}$-$NH_2$

AP168 (des$NH_2$-F)-G-G-R-I-D-R-I-G-$A_{\dagger}$-$NH_2$

AP169 A-F-G-G-R-I-D-R-I-$A^{\dagger}$-A-$NH_2$

AP170 F-G-G-R-I-D-R-I-$A_{\dagger}$-A-$NH_2$

AP171 (des$NH_2$-F)-G-G-R-I-D-R-I-$A_{\dagger}$-A-$NH_2$

| | |
|---|---|
| AP172 | A-F-G-G-R-I-D-R-I-$A^{\dagger}$-$NH_2$ |
| AP173 | F-G-G-R-I-D-R-I-$A^{\dagger}$-$NH_2$ |
| AP174 | (des$NH_2$-F)-G-G-R-I-D-R-I-$A^{\dagger}$-$NH_2$ |
| AP175 | Y-A-F-G-G-R-I-D-R-I-G-A-$NH_2$ |
| AP176 | A-F-G-G-R-I-D-R-I-G-Y-$NH_2$ |
| AP177 | A-F-G-G-R-I-E-R-I-G-A-$NH_2$ |
| AP178 | A-F-G-G-K-I-E-R-I-G-A-$NH_2$ |
| AP179 | A-F-G-G-K-I-D-R-I-G-A-$NH_2$ |
| AP180 | A-F-G-G-R-I-D-K-I-G-A-$NH_2$ |
| AP181 | (des$NH_2$-F)-G-G-R-M-D-R-I-G-A-$NH_2$ |
| AP182 | (des$NH_2$-F)-G-G-R-M-D-R-I-G-$NH_2$ |
| AP183 | (des$NH_2$-F)-G-G-R-M-D-R-I-$NH_2$ |
| AP184 | A-F-$A^{\dagger}$-G-R-M-D-R-I-G-A-$NH_2$ |
| AP185 | A-F-$A^{\dagger}$-G-R-M-D-R-I-G-$NH_2$ |
| AP186 | A-F-$A^{\dagger}$-G-R-M-D-R-I-$NH_2$ |
| AP187 | F-$A^{\dagger}$-G-R-M-D-R-I-G-A-$NH_2$ |
| AP188 | F-$A^{\dagger}$-G-R-M-D-R-I-G-$NH_2$ |
| AP189 | F-$A^{\dagger}$-G-R-M-D-R-I-$NH_2$ |

AP190 (desNH$_2$-F)-A$^†$-G-R-M-D-R-I-G-A-NH$_2$

AP191 (desNH$_2$-F)-A$^†$-G-R-M-D-R-I-G-NH$_2$

AP192 (desNH$_2$-F)-A$^†$-G-R-M-D-R-I-NH$_2$

AP193 A-F-S$^†$-G-R-M-D-R-I-G-A-NH$_2$

AP194 A-F-S$^†$-G-R-M-D-R-I-G-NH$_2$

AP195 A-F-S$^†$-G-R-M-D-R-I-NH$_2$

AP196 F-S$^†$-G-R-M-D-R-I-G-A-NH$_2$

AP197 F-S$^†$-G-R-M-D-R-I-G-NH$_2$

AP198 F-S$^†$-G-R-M-D-R-I-NH$_2$

AP199 (desNH$_2$-F)-S$^†$-G-R-M-D-R-I-G-A-NH$_2$

AP200 (desNH$_2$-F)-S$^†$-G-R-M-D-R-I-G-NH$_2$

AP201 (desNH$_2$-F)-S$^†$-G-R-M-D-R-I-NH$_2$

AP202 A-F$^†$-G-G-R-M-D-R-I-G-A-NH$_2$

AP203 A-F$^†$-G-G-R-M-D-R-I-G-NH$_2$

AP204 A-F$^†$-G-G-R-M-D-R-I-NH$_2$

AP205 F$^†$-G-G-R-M-D-R-I-G-A-NH$_2$

AP206 F$^†$-G-G-R-M-D-R-I-G-NH$_2$

AP207 F$^†$-G-G-R-M-D-R-I-NH$_2$

| | |
|---|---|
| AP208 | A-F-G-A$^{\dagger}$-R-M-D-R-I-G-A-$NH_2$ |
| AP209 | A-F-G-A$^{\dagger}$-R-M-D-R-I-G-$NH_2$ |
| AP210 | A-F-G-A$^{\dagger}$-R-M-D-R-I-$NH_2$ |
| AP211 | F-G-A$^{\dagger}$-R-M-D-R-I-G-A-$NH_2$ |
| AP212 | F-G-A$^{\dagger}$-R-M-D-R-I-G-$NH_2$ |
| AP213 | F-G-A$^{\dagger}$-R-M-D-R-I-$NH_2$ |
| AP214 | (des$NH_2$-F)-G-A$^{\dagger}$-R-M-D-R-I-G-A-$NH_2$ |
| AP215 | (des$NH_2$-F)-G-A$^{\dagger}$-R-M-D-R-I-G-$NH_2$ |
| AP216 | (des$NH_2$-F)-G-A$^{\dagger}$-R-M-D-R-I-$NH_2$ |
| AP217 | A-F-G-G-R-M-D-R-I-G-A$^{\dagger}$-$NH_2$ |
| AP218 | F-G-G-R-M-D-R-I-G-A$^{\dagger}$-$NH_2$ |
| AP219 | (des$NH_2$-F)-G-G-R-M-D-R-I-G-A$^{\dagger}$-$NH_2$ |
| AP220 | A-F-G-G-R-M-D-R-I-A$^{\dagger}$-A-$NH_2$ |
| AP221 | F-G-G-R-M-D-R-I-A$^{\dagger}$-A-$NH_2$ |
| AP222 | (des$NH_2$-F)-G-G-R-M-D-R-I-A$^{\dagger}$-A-$NH_2$ |
| AP223 | A-F-G-G-R-M-D-R-I-A$^{\dagger}$-$NH_2$ |
| AP224 | F-G-G-R-M-D-R-I-A$^{\dagger}$-$NH_2$ |
| AP225 | (des$NH_2$-F)-G-G-R-M-D-R-I-A$^{\dagger}$-$NH_2$ |

AP226 Y-A-F-G-G-R-M-D-R-I-G-A-$NH_2$

AP227 A-F-G-G-R-M-D-R-I-G-Y-$NH_2$

AP228 A-F-G-G-R-M-E-R-I-G-A-$NH_2$

AP229 A-F-G-G-K-M-E-R-I-G-A-$NH_2$

AP230 A-F-G-G-K-M-D-R-I-G-A-$NH_2$

AP231 A-F-G-G-R-M-D-K-I-G-A-$NH_2$

AP290 F-G-G-R-M-D-R-I-$NH_2$

AP293 F-G-G-R-M-D-R-I-G-$NH_2$

AP297 F-G-G-R-M-D-R-I-G-A-$NH_2$

* AP302 A-F-G-G-R-$I^{\dagger}$-D-R-I-G-A-$NH_2$

* AP304 A-F-G-G-R-I-D-$R^{\dagger}$-I-G-A-$NH_2$

* AP305 A-F-G-G-R-I-D-R-$I^{\dagger}$-G-A-$NH_2$

Part B

* AP306 (2-NA)-G-G-R-I-D-R-I-G-A-$NH_2$

* AP307 (2-NOA)-$NH(CH_2)_4CO$-R-I-D-R-I-$NH_2$

* AP308 (TPP)-G-G-R-I-D-R-I-$NH_2$

* AP309 (1-AA)-G-G-R-I-D-R-I-$NH_2$

* AP310 (CBZ)-G-G-R-I-D-R-I-$NH_2$

* AP311 (FMOC)-G-G-R-I-D-R-I-$NH_2$

* AP312 (FMOC)-G-G-R-I-D-R-I-G-A-$NH_2$

* AP313 (IP)-G-G-R-I-D-R-I-G-A-$NH_2$

* AP314 (2-NA)-G-G-R-I-D-R-I-$NH_2$

* AP315 (1-NA)-G-G-R-I-D-R-I-G-A-$NH_2$

* AP316 (1-NOA)-G-G-R-I-D-R-I-$NH_2$

* AP317 (4-BPA)-G-G-R-I-D-R-I-$NH_2$

* AP318 (2-NOA)-G-G-R-I-D-R-I-G-A-$NH_2$

* AP319 (2-NOA)-G-G-R-I-D-R-I-$NH_2$

* AP320 (2-NA)-G-(Sar)-R-I-D-R-I-$NH_2$

* AP321 (2-NA)-G-(Aib)-R-I-D-R-I-$NH_2$

* AP322 (2-NOA)-$A^{\dagger}$-G-R-I-D-R-I-$NH_2$

* AP323 (FMOC)-$NH(CH_2)_4CO$-R-I-D-R-I-$NH_2$

* AP324 (2-NA)-$NH(CH_2)_4CO$-R-I-D-R-I-$NH_2$

* AP325 (2-NOA)-$NH(CH_2)_5CO$-R-I-D-R-I-$NH_2$

* AP326 (FMOC)-$NH(CH_2)_3CO$-R-I-D-R-I-$NH_2$

* AP327 (2-NA)-$NH(CH_2)_3CO$-R-I-D-R-I-$NH_2$

* AP328 (2-NA)-G-G-R-I-D-R-I-$NHCH_2CH_3$

| | | |
|---|---|---|
| * | AP329 | (2-NA)-NH$(CH_2)_5$CO-R-I-D-R-I-$NH_2$ |
| * | AP330 | (2-NA)-S[T]-G-R-I-D-R-I-$NH_2$ |
| * | AP331 | (3-DPP)-G-G-R-I-D-R-I-G-A-$NH_2$ |
| * | AP332 | (2-NA)-G-G-R-I-D-R-L-$NH_2$ |
| * | AP333 | (2-NA)-G-G-R-I-D-R-V-$NH_2$ |
| * | AP334 | (CHA)-G-G-R-I-D-R-I-$NH_2$ |
| | AP335 | (2-NOA)-G-G-R-M-D-Q-I-G-A-$NH_2$ |
| | AP336 | (2-NOA)-G-G-R-M-D-Q-I-G-$NH_2$ |
| * | AP337 | (2-NOA)-G-G-R-M-D-Q-I-$NH_2$ |
| | AP338 | (2-NOA)-G-G-A-I-D-R-I-$NH_2$ |
| | AP339 | (2-NOA)-G-G-A-I-D-R-I-G-$NH_2$ |
| | AP340 | (2-NOA)-G-G-A-I-D-R-I-G-A-$NH_2$ |
| | AP341 | (2-NOA)-G-G-A-M-D-R-I-G-A-$NH_2$ |
| | AP342 | (2-NOA)-G-G-A-M-D-R-I-G-$NH_2$ |
| | AP343 | (2-NOA)-G-G-A-M-D-R-I-$NH_2$ |
| | AP344 | (2-NA)-G-G-R-M-D-R-V-$NH_2$ |
| | AP345 | (2-NA)-G-G-R-M-D-R-L-$NH_2$ |
| | AP346 | (2-NA)-G-G-R-M-D-R-M-$NH_2$ |

AP347 (2-NA)-G-G-R-V-D-R-I-$NH_2$

AP348 (2-NA)-G-G-R-V-D-R-V-$NH_2$

AP349 (2-NA)-G-G-R-V-D-R-L-$NH_2$

AP350 (2-NA)-G-G-R-V-D-R-M-$NH_2$

* AP351 (2-NA)-G-G-R-I-D-Q-I-$NH_2$

AP352 (2-NA)-G-G-R-I-D-Q-I-G-$NH_2$

AP353 (2-NA)-G-G-R-I-D-Q-I-G-A-$NH_2$

AP354 (2-NA)-G-G-R-M-D-Q-I-G-A-$NH_2$

AP355 (2-NA)-G-G-R-M-D-Q-I-G-$NH_2$

AP356 (2-NA)-G-G-R-M-D-Q-I-$NH_2$

* AP357 (2-NA)-G-G-A-I-D-R-I-$NH_2$

AP358 (2-NA)-G-G-A-I-D-R-I-G-$NH_2$

AP359 (2-NA)-G-G-A-I-D-R-I-G-A-$NH_2$

AP360 (2-NA)-G-G-A-M-D-R-I-G-A-$NH_2$

AP361 (2-NA)-G-G-A-M-D-R-I-G-$NH_2$

AP362 (2-NA)-G-G-A-M-D-R-I-$NH_2$

AP363 (2-NOA)-G-G-R-I-D-R-V-$NH_2$

AP364 (2-NOA)-G-G-R-M-D-R-V-$NH_2$

* AP365 (2-NOA)-G-G-R-I-D-R-L-$NH_2$

AP366 (2-NOA)-G-G-R-I-D-R-M-$NH_2$

AP367 (2-NOA)-G-G-R-M-D-R-M-$NH_2$

AP368 (2-NOA)-G-G-R-M-D-R-L-$NH_2$

AP369 (2-NOA)-G-G-R-I-D-Q-I-$NH_2$

AP370 (2-NOA)-G-G-R-I-D-Q-I-G-$NH_2$

AP371 (2-NOA)-G-G-R-I-D-Q-I-G-A-$NH_2$

AP374 (2-NOA)-G-(Aib)-R-I-D-R-I-$NH_2$

AP375 (2-NOA)-G-(Aib)-R-I-D-R-I-G-$NH_2$

AP376 (2-NOA)-G-(Aib)-R-I-D-R-I-G-A-$NH_2$

AP377 (2-NOA)-G-(Aib)-R-M-D-R-I-G-A-$NH_2$

AP378 (2-NOA)-G-(Aib)-R-M-D-R-I-G-$NH_2$

AP379 (2-NOA)-G-(Aib)-R-M-D-R-I-$NH_2$

AP380 (2-NOA)-G-(Sar)-R-I-D-R-I-G-$NH_2$

AP381 (2-NOA)-G-(Sar)-R-I-D-R-I-$NH_2$

AP382 (2-NOA)-G-(Sar)-R-I-D-R-I-G-A-$NH_2$

AP383 (2-NOA)-G-(Sar)-R-M-D-R-I-G-A-$NH_2$

AP384 (2-NOA)-G-(Sar)-R-M-D-R-I-G-$NH_2$

AP385 (2-NOA)-G-(Sar)-R-M-D-R-I-$NH_2$

AP386 (2-NA)-G-(Sar)-R-I-D-R-I-G-$NH_2$

AP387 (2-NA)-G-(Sar)-R-I-D-R-I-G-A-$NH_2$

AP388 (2-NA)-G-(Sar)-R-M-D-R-I-G-A-$NH_2$

AP389 (2-NA)-G-(Sar)-R-M-D-R-I-G-$NH_2$

* AP390 (2-NA)-G-(Sar)-R-M-D-R-I-$NH_2$

AP391 (2-NOA)-G-G-R-I-D-R-I-$NHCH_2CH_3$

AP393 (2-NOA)-G-G-R-I-D-R-I-G-$NH_2$

AP394 (2-NOA)-G-G-R-M-D-R-I-G-A-$NH_2$

AP395 (2-NOA)-G-G-R-M-D-R-I-G-$NH_2$

AP396 (2-NOA)-G-G-R-M-D-R-I-$NH_2$

AP397 (2-NOA)-$S^†$-G-R-M-D-R-I-G-$NH_2$

AP398 (2-NOA)-$S^†$-G-R-M-D-R-I-$NH_2$

AP399 (2-NOA)-G-$A^†$-R-I-D-R-I-$NH_2$

AP400 (2-NOA)-G-$A^†$-R-I-D-R-I-G-$NH_2$

AP401 (2-NOA)-G-$A^†$-R-M-D-R-I-G-A-$NH_2$

AP402 (2-NOA)-G-$A^†$-R-M-D-R-I-G-$NH_2$

AP403 (2-NOA)-G-$A^†$-R-M-D-R-I-$NH_2$

* AP404 (2-NA)-G-A† -R-I-D-R-I-$NH_2$

AP405 (2-NA)-G-A† -R-I-D-R-I-G-$NH_2$

AP406 (2-NA)-G-A† -R-I-D-R-I-G-A-$NH_2$

AP407 (2-NA)-G-A† -R-M-D-R-I-G-A-$NH_2$

AP408 (2-NA)-G-A† -R-M-D-R-I-G-$NH_2$

AP409 (2-NA)-G-A† -R-M-D-R-I-$NH_2$

AP410 (2-NA)-G-(Aib)-R-I-D-R-I-G-$NH_2$

AP411 (2-NA)-G-(Aib)-R-I-D-R-I-G-A-$NH_2$

AP412 (2-NA)-G-(Aib)-R-M-D-R-I-G-A-$NH_2$

AP413 (2-NA)-G-(Aib)-R-M-D-R-I-G-$NH_2$

* AP414 (2-NA)-G-(Aib)-R-M-D-R-I-$NH_2$

* AP415 (2-NOA)-$NH(CH_2)_3CO$-R-I-D-R-I-$NH_2$

AP416 (2-NOA)-$NH(CH_2)_3CO$-R-I-D-R-I-G-$NH_2$

AP417 (2-NOA)-$NH(CH_2)_3CO$-R-I-D-R-I-G-A-$NH_2$

AP418 (2-NOA)-$NH(CH_2)_3CO$-R-M-D-R-I-G-A-$NH_2$

AP419 (2-NOA)-$NH(CH_2)_3CO$-R-M-D-R-I-G-$NH_2$

AP420 (2-NOA)-$NH(CH_2)_3CO$-R-M-D-R-I-$NH_2$

AP421 (2-NOA)-$NH(CH_2)_5CO$-R-I-D-R-I-G-$NH_2$

AP422 (2-NOA)-NH$(CH_2)_5$CO-R-I-D-R-I-G-A-$NH_2$

AP423 (2-NOA)-NH$(CH_2)_5$CO-R-M-D-R-I-G-A-$NH_2$

AP424 (2-NOA)-NH$(CH_2)_5$CO-R-M-D-R-I-G-$NH_2$

AP425 (2-NOA)-NH$(CH_2)_5$CO-R-M-D-R-I-$NH_2$

AP426 (2-NA)-NH$(CH_2)_5$CO-R-M-D-R-I-$NH_2$

AP427 (2-NA)-NH$(CH_2)_5$CO-R-M-D-R-I-G-$NH_2$

AP428 (2-NA)-NH$(CH_2)_5$CO-R-M-D-R-I-G-A-$NH_2$

* AP429 (2-NA)-NH$(CH_2)_5$CO-R-I-D-R-I-G-A-$NH_2$

AP430 (2-NA)-NH$(CH_2)_5$CO-R-I-D-R-I-G-$NH_2$

AP431 (2-NA)-S$^{\dagger}$-G-R-I-D-R-I-G-$NH_2$

AP432 (2-NA)-S$^{\dagger}$-G-R-I-D-R-I-G-A-$NH_2$

AP433 (2-NA)-S$^{\dagger}$-G-R-M-D-R-I-G-A-$NH_2$

AP434 (2-NA)-S$^{\dagger}$-G-R-M-D-R-I-G-$NH_2$

AP435 (2-NA)-S$^{\dagger}$-G-R-M-D-R-I-$NH_2$

AP436 (2-NOA)-S$^{\dagger}$-G-R-I-D-R-I-$NH_2$

AP437 (2-NOA)-S$^{\dagger}$-G-R-I-D-R-I-G-$NH_2$

AP438 (2-NOA)-S$^{\dagger}$-G-R-I-D-R-I-G-A-$NH_2$

AP439 (2-NOA)-S$^{\dagger}$-G-R-M-D-R-I-G-A-$NH_2$

* AP440 (2-NA)-A$^{\dagger}$-G-R-I-D-R-I-$NH_2$

AP441 (2-NA)-A$^{\dagger}$-G-R-I-D-R-I-G-$NH_2$

AP442 (2-NA)-A$^{\dagger}$-G-R-I-D-R-I-G-A-$NH_2$

AP443 (2-NA)-A$^{\dagger}$-G-R-M-D-R-I-G-A-$NH_2$

AP444 (2-NA)-A$^{\dagger}$-G-R-M-D-R-I-G-$NH_2$

AP445 (2-NA)-A$^{\dagger}$-G-R-M-D-R-I-$NH_2$

AP446 (2-NOA)-A$^{\dagger}$-G-R-I-D-R-I-G-$NH_2$

AP447 (2-NOA)-A$^{\dagger}$-G-R-I-D-R-I-G-A-$NH_2$

AP448 (2-NOA)-A$^{\dagger}$-G-R-M-D-R-I-G-A-$NH_2$

AP449 (2-NOA)-A$^{\dagger}$-G-R-M-D-R-I-G-$NH_2$

AP450 (2-NOA)-A$^{\dagger}$-G-R-M-D-R-I-$NH_2$

AP451 (2-NA)-$NH(CH_2)_3CO$-R-I-D-R-I-G-$NH_2$

AP452 (2-NA)-$NH(CH_2)_3CO$-R-I-D-R-I-G-A-$NH_2$

AP453 (2-NA)-$NH(CH_2)_3CO$-R-M-D-R-I-G-A-$NH_2$

AP454 (2-NA)-$NH(CH_2)_3CO$-R-M-D-R-I-G-$NH_2$

AP455 (2-NA)-$NH(CH_2)_3CO$-R-M-D-R-I-$NH_2$

AP456 (2-NOA)-$NH(CH_2)_4CO$-R-I-D-R-I-G-$NH_2$

AP457 (2-NOA)-$NH(CH_2)_4CO$-R-I-D-R-I-G-A-$NH_2$

AP458 (2-NOA)-NH$(CH_2)_4$CO-R-M-D-R-I-G-$NH_2$

* AP459 (2-NOA)-NH$(CH_2)_4$CO-R-M-D-R-I-$NH_2$

AP460 (2-NOA)-NH$(CH_2)_4$CO-R-I-D-R-V-$NH_2$

AP461 (2-NOA)-NH$(CH_2)_4$CO-R-I-D-R-L-$NH_2$

AP462 (2-NOA)-NH$(CH_2)_4$CO-R-I-D-R-M-$NH_2$

AP463 (2-NOA)-NH$(CH_2)_4$CO-R-M-D-R-M-$NH_2$

AP464 (2-NOA)-NH$(CH_2)_4$CO-R-M-D-R-V-$NH_2$

AP465 (2-NOA)-NH$(CH_2)_4$CO-R-M-D-R-L-$NH_2$

AP466 (2-NOA)-NH$(CH_2)_4$CO-R-L-D-R-L-$NH_2$

AP467 (2-NOA)-NH$(CH_2)_4$CO-R-L-D-R-M-$NH_2$

AP468 (2-NOA)-NH$(CH_2)_4$CO-R-L-D-R-V-$NH_2$

AP469 (2-NOA)-NH$(CH_2)_4$CO-R-L-D-R-I-$NH_2$

AP470 (2-NOA)-NH$(CH_2)_4$CO-R-V-D-R-I-$NH_2$

AP471 (2-NOA)-NH$(CH_2)_4$CO-R-V-D-R-V-$NH_2$

AP472 (2-NOA)-NH$(CH_2)_4$CO-R-V-D-R-L-$NH_2$

AP473 (2-NOA)-NH$(CH_2)_4$CO-R-V-D-R-M-$NH_2$

AP474 (2-NOA)-NH$(CH_2)_4$CO-A-I-D-R-I-$NH_2$

AP475 (2-NOA)-NH$(CH_2)_4$CO-A-M-D-R-I-$NH_2$

AP476 (2-NOA)-NH$(CH_2)_4$CO-R-I-D-Q-I-$NH_2$

AP477 (2-NOA)-NH$(CH_2)_4$CO-R-I-D-Q-I-G-$NH_2$

AP478 (2-NOA)-NH$(CH_2)_4$CO-R-I-D-Q-I-G-A-$NH_2$

AP479 (2-NOA)-NH$(CH_2)_4$CO-R-M-D-Q-I-G-A-$NH_2$

AP480 (2-NOA)-NH$(CH_2)_4$CO-R-M-D-Q-I-G-$NH_2$

AP481 (2-NOA)-NH$(CH_2)_4$CO-R-M-D-Q-I-$NH_2$

AP482 (2-NA)-NH$(CH_2)_4$CO-R-I-D-R-I-G-$NH_2$

* AP483 (2-NA)-NH$(CH_2)_4$CO-R-I-D-R-I-G-A-$NH_2$

AP484 (2-NA)-NH$(CH_2)_4$CO-R-M-D-R-I-G-A-$NH_2$

AP485 (2-NA)-NH$(CH_2)_4$CO-R-M-D-R-I-G-$NH_2$

* AP486 (2-NA)-NH$(CH_2)_4$CO-R-M-D-R-I-$NH_2$

AP487 (2-NA)-NH$(CH_2)_4$CO-R-I-D-R-V-$NH_2$

* AP488 (2-NA)-NH$(CH_2)_4$CO-R-I-D-R-L-$NH_2$

AP489 (2-NA)-NH$(CH_2)_4$CO-R-I-D-R-M-$NH_2$

AP490 (2-NA)-NH$(CH_2)_4$CO-R-M-D-R-M-$NH_2$

AP491 (2-NA)-NH$(CH_2)_4$CO-R-M-D-R-L-$NH_2$

AP492 (2-NA)-NH$(CH_2)_4$CO-R-M-D-R-V-$NH_2$

AP493 (2-NA)-NH$(CH_2)_4$CO-A-I-D-R-I-$NH_2$

AP494 $(2\text{-NA})\text{-NH}(CH_2)_4CO\text{-A-M-D-R-I-NH}_2$

AP495 $(2\text{-NA})\text{-NH}(CH_2)_4CO\text{-R-I-D-Q-I-NH}_2$

AP496 $(2\text{-NA})\text{-NH}(CH_2)_4CO\text{-R-M-D-Q-I-NH}_2$

AP497 $(2\text{-NA})\text{-NH}(CH_2)_4CO\text{-R-I-D-Q-I-G-NH}_2$

AP498 $(2\text{-NA})\text{-NH}(CH_2)_4CO\text{-R-I-D-Q-I-G-A-NH}_2$

AP499 $(2\text{-NA})\text{-NH}(CH_2)_4CO\text{-R-M-D-Q-I-G-A-NH}_2$

AP500 $(2\text{-NA})\text{-NH}(CH_2)_4CO\text{-R-M-D-Q-I-G-NH}_2$

* AP501 $(2\text{-NA})\text{-NH}(CH_2)_4CO\text{-R-L-D-R-I-NH}_2$

AP502 $(2\text{-NA})\text{-NH}(CH_2)_4CO\text{-R-L-D-R-M-NH}_2$

AP503 $(2\text{-NA})\text{-NH}(CH_2)_4CO\text{-R-L-D-R-V-NH}_2$

AP504 $(2\text{-NA})\text{-NH}(CH_2)_4CO\text{-R-L-D-R-L-NH}_2$

AP505 $(2\text{-NA})\text{-NH}(CH_2)_4CO\text{-R-V-D-R-I-NH}_2$

AP506 $(2\text{-NA})\text{-NH}(CH_2)_4CO\text{-R-V-D-R-L-NH}_2$

AP507 $(2\text{-NA})\text{-NH}(CH_2)_4CO\text{-R-V-D-R-V-NH}_2$

AP508 $(2\text{-NA})\text{-NH}(CH_2)_4CO\text{-R-V-D-R-M-NH}_2$

AP509 $(2\text{-NOA})\text{-G-G-R-I-D-R-NH-}CH_2\text{-}CH(CH_3)_2$

AP510 $(2\text{-NA})\text{-G-G-R-I-D-R-NH-}CH_2\text{-}CH(CH_3)_2$

AP511 $(2\text{-NA})\text{-G-G-R-I-D-R-NH-}CH_2\text{-}CH(CH_3)CH_2CH_3$

AP517 (2-NA)-G-G-R-I-D-$Q^{\dagger}$-I-$NH_2$

AP518 (2-NA)-G-G-R-I-E-R-I-$NH_2$

AP522 (2-NA)-G-G-Q-I-D-R-I-$NH_2$

AP523 (2-NA)-G-G-K-I-D-R-I-$NH_2$

* AP524 (2-NA)-G-G-R-I-D-R-I-G-NH-(ethyl)

* AP525 (2-NA)-G-G-R-I-D-R-I-G-(Aib)-$NH_2$

* AP526 (2-NA)-G-G-R-I-D-R-I-(5-aminovaleryl)-$NH_2$

* AP527 (2-NA)-G-G-R-(Cha)-D-R-I-$NH_2$

* AP528 2-(2'-(6'-MeONAP))-G-G-R-I-D-R-I-G-A-$NH_2$

* AP529 (2-NA)-G-G-R-I-D-R-(N-MeIle)-G-NH-(ethyl)

* AP530 (2-NA)-$NH(CH_2)_4CO$-R-I-D-R-I-G-NH-(ethyl)

* AP531 (3-IB)-G-R-I-D-R-I-G-A-$NH_2$)

* AP532 (3-IB)-beta-Ala-R-I-D-R-I-G-A-$NH_2$

* AP533 (bis-(1'-NM)acetyl)-G-G-R-I-D-R-I-G-A-$NH_2$

* AP534 (DBA)-G-G-R-I-D-R-I-G-A-$NH_2$

* AP535 (2-NA)-G-G-R-I-D-R-$V^{\dagger}$-$NH_2$

* AP536 (2-NA)-G-G-R-I-D-R-$L^{\dagger}$-$NH_2$

* AP538 (2-NA)-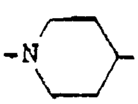-CO-R-I-D-R-I-$NH_2$

* AP539 (2-NOA)-N 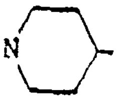 -CO-R-I-D-R-I-$NH_2$

* AP557 (2-NA)-N 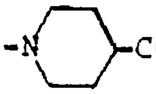 -CO-R-I-D-R-NH-(2-methylbutyl)

* AP560 (2-NA)-NH- 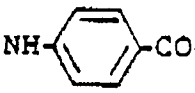 -CO-R-I-D-R-I-$NH_2$

AP561 (2-NTA)-N 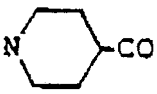 -CO-R-I-D-R-I-$NH_2$

* AP562 (2-NYL)-N 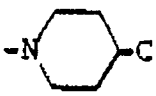 -CO-R-I-D-R-I-$NH_2$

AP563 (3-POP)-N 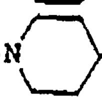 -CO-R-I-D-R-I-$NH_2$

* AP564 (2-NA)-NH- 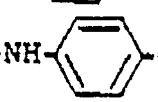 -$CH_2$-CO-R-I-D-R-I-$NH_2$

AP565 (2-NA)-NH-$CH_2$- 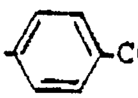 -CO-R-I-D-R-I-$NH_2$

AP566 (2-NA)-NH- 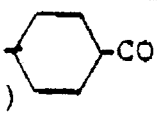 -CO-R-I-D-R-I-$NH_2$ (cis and trans)

AP567 (2-NOA)-NH- 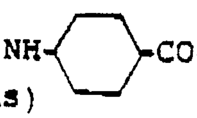 -CO-R-I-D-R-I-$NH_2$ (cis and trans)

* AP568 (2-NA)-NH-$CH_2$- 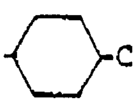 -CO-R-I-D-R-I-$NH_2$ (trans)

AP569 (2-NOA)-NH-$CH_2$- 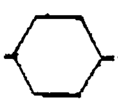 -CO-R-I-D-R-I-$NH_2$ (trans)

AP570 (2-NL)-$CH_2$-$CH_2$-N 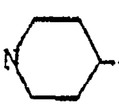 -CO-R-I-D-R-I-$NH_2$

AP571 (2-NO)-$CH_2CH_2$-N 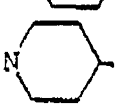 -CO-R-I-D-R-I-$NH_2$

AP572 (2-NYL)-NH-$CH_2$- 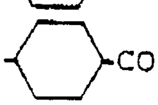 -CO-R-I-D-R-I-$NH_2$ (trans)

AP573 (2-NL)-$CH_2$-NH-$CH_2$-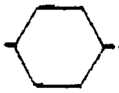-CO-R-I-D-R-I-$NH_2$

* AP574 (2-NYL)-$CH_2$-N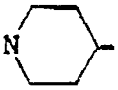-CO-R-I-D-R-I-$NH_2$

* AP576 (2-NOA)-NH$(CH_2)_3$CO-R-I-D-R-I-G-A-$NH_2$

* AP590 (2-NA)-G-G-R-tBuA-D-R-I-$NH_2$

* AP591 (2-NA)-G-G-R-tBuG-D-R-I-$NH_2$

* AP592 (2-NA)-G-G-R-I-D-Orn-I-$NH_2$

* AP593 (2-NA)-G-G-R-I-D-Cit-I-$NH_2$

* AP594 (2-NA)-G-G-R-I-D-R-tBuA-$NH_2$

* AP596 (2-NA)-G-G-R-I-D-R-NMeIle-NH(ethyl)

* AP597 (3-1B)-G-R-I-D-R-I-G-A-$NH_2$

AP598 (3-1B)-βala-R-I-D-R-I-G-A-$NH_2$

* AP599 (2-NA)-G-G-R-Cha-D-R-I-$NH_2$

AP700 2-NA-NH-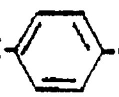-$CH_2$-CO-R($Ch_2$-NH)I-D-R-I-$NH_2$

AP701 2-NA-NH-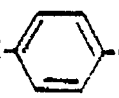-$CH_2$-CO-R-I($CH_2$-NH)D-R-I-$NH_2$

AP702 2-NA-NH-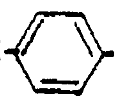-$CH_2$-CO-R-I-D($CH_2$-NH)R-I-$NH_2$

AP703 2-NA-NH-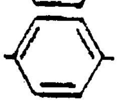-$CH_2$-CO-R-I-D-R($CH_2$-NH)I-$NH_2$

* AP704 2-NA-N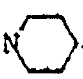-$CH_2$-R-I-D-R-I-$NH_2$

AP705 (2-NA)-G-G-R-I-D-R-I-G-$NH_2$

AP706 (2-NA)-G-G-R-M-D-R-I-$NH_2$

AP707 (2-NA)-G-G-R-M-D-R-I-G-$NH_2$

AP708 (2-NA)-G-G-R-M-D-R-I-G-A-$NH_2$

AP709 (TPP)-G-G-R-I-D-R-I-G-$NH_2$

AP710 (TPP)-G-G-R-I-D-R-I-G-A-$NH_2$

AP711 (TPP)-G-G-R-M-D-R-I-$NH_2$

AP712 (TPP)-G-G-R-M-D-R-I-G-$NH_2$

AP713 (TPP)-G-G-R-M-D-R-I-G-A-$NH_2$

AP714 (1-AA)-G-G-R-I-D-R-I-G-$NH_2$

AP715 (1-AA)-G-G-R-I-D-R-I-G-A-$NH_2$

AP716 (1-AA)-G-G-R-M-D-R-I-$NH_2$

AP717 (1-AA)-G-G-R-M-D-R-I-G-$NH_2$

AP718 (1-AA)-G-G-R-M-D-R-I-G-A-$NH_2$

AP719 (CBZ)-G-G-R-I-D-R-I-G-$NH_2$

AP720 (CBZ)-G-G-R-I-D-R-I-G-A-$NH_2$

AP721 (CBZ)-G-G-R-M-D-R-I-$NH_2$

AP722 (CBZ)-G-G-R-M-D-R-I-G-$NH_2$

AP723 (CBZ)-G-G-R-M-D-R-I-G-A-$NH_2$

AP724 (FMOC)-G-G-R-I-D-R-I-G-$NH_2$

AP725 (FMOC)-G-G-R-M-D-R-I-$NH_2$

AP726 (FMOC)-G-G-R-M-D-R-I-G-$NH_2$

AP727 (FMOC)-G-G-R-M-D-R-I-G-A-$NH_2$

AP728 (IP)-G-G-R-I-D-R-I-G-$NH_2$

AP729 (IP)-G-G-R-I-D-R-I-G-A-$NH_2$

AP730 (IP)-G-G-R-M-D-R-I-$NH_2$

AP731 (IP)-G-G-R-M-D-R-I-G-$NH_2$

AP732 (IP)-G-G-R-M-D-R-I-G-A-$NH_2$

AP733 (1-NA)-G-G-R-I-D-R-I-$NH_2$

AP734 (1-NA)-G-G-R-I-D-R-I-G-$NH_2$

AP735 (1-NA)-G-G-R-M-D-R-I-$NH_2$

AP736 (1-NA)-G-G-R-M-D-R-I-G-$NH_2$

AP737 (1-NA)-G-G-R-M-D-R-I-G-A-$NH_2$

AP738 (1-NOA)-G-G-R-I-D-R-I-G-$NH_2$

AP739 (1-NOA)-G-G-R-I-D-R-I-G-A-$NH_2$

AP740 (1-NOA)-G-G-R-M-D-R-I-$NH_2$

AP741 (1-NOA)-G-G-R-M-D-R-I-G-$NH_2$

AP741 (1-NOA)-G-G-R-M-D-R-I-G-A-$NH_2$

AP734 (4-BPA)-G-G-R-I-D-R-I-G-$NH_2$

AP744 (4-BPA)-G-G-R-I-D-R-I-G-A-$NH_2$

AP745 (4-BPA)-G-G-R-M-D-R-I-$NH_2$

AP746 (4-BPA)-G-G-R-M-D-R-I-G-$NH_2$

AP747 (4-BPA)-G-G-R-M-D-R-I-G-A-$NH_2$

AP748 (FMOC)-$NH(CH_2)_4CO$-R-I-D-R-I-G-$NH_2$

AP749 (FMOC)-$NH(CH_2)_4CO$-R-I-D-R-I-G-A-$NH_2$

AP750 (FMOC)-$NH(CH_2)_4CO$-R-M-D-R-I-$NH_2$

AP751 (FMOC)-$NH(CH_2)_4CO$-R-M-D-R-I-G-$NH_2$

AP752 (FMOC)-$NH(CH_2)_4CO$-R-M-D-R-I-G-A-$NH_2$

AP753 (3-DPP)-$NH(CH_2)_3CO$-R-I-D-R-I-G-$NH_2$

AP754 (3-DPP)-$NH(CH_2)_3CO$-R-I-D-R-I-G-A-$NH_2$

AP755 (3-DPP)-$NH(CH_2)_3CO$-R-M-D-R-I-$NH_2$

AP756 (3-DPP)-$NH(CH_2)_3CO$-R-M-D-R-I-G-$NH_2$

AP757 (3-DPP)-$NH(CH_2)_3CO$-R-M-D-R-I-G-A-$NH_2$

AP758 (CHA)-G-G-R-I-D-R-I-G-$NH_2$

AP759 (CHA)-G-G-R-I-D-R-I-G-A-$NH_2$

AP760 (CHA)-G-G-R-M-D-R-I-$NH_2$

AP761 (CHA)-G-G-R-M-D-R-I-G-$NH_2$

AP762 (CHA)-G-G-R-M-D-R-I-G-A-$NH_2$

AP763 (2-NA)-G-G-L-M-D-R-I-$NH_2$

AP764 (2-NA)-G-G-L-M-D-R-L-$NH_2$

AP765 (2-NA)-G-G-L-M-D-R-M-$NH_2$

AP766 (2-NA)-G-G-L-M-D-R-V-$NH_2$

AP767 (2-NA)-G-G-I-M-D-R-M-$NH_2$

AP768 (2-NOA)-G-G-L-M-D-R-I-$NH_2$

AP769 (2-NOA)-G-G-L-M-D-R-L-$NH_2$

AP770 (2-NOA)-G-G-L-M-D-R-M-$NH_2$

AP771 (2-NOA)-G-G-L-M-D-R-V-$NH_2$

AP772 (2-NOA)-G-G-I-V-D-R-M-$NH_2$

AP773 (2-NOA)-G-G-I-V-D-R-I-$NH_2$

AP774 (2-NOA)-G-G-I-V-D-R-L-$NH_2$

AP775 (2-NOA)-G-G-I-V-D-R-V-$NH_2$

AP776 (2-NOA)-G-A† -R-I-D-R-I-G-A-$NH_2$

AP777 (2-NOA)-NH$(CH_2)_4$CO-R-M-D-R-I-G-A-$NH_2$

AP778 (2-NOA)-NH$(CH_2)_4$CO-A-I-D-R-I-$NH_2$

AP779 (2-NOA)-NH$(CH_2)_4$CO-A-I-D-R-I-G-$NH_2$

AP780 (2-NOA)-NH$(CH_2)_4$CO-A-I-D-R-I-G-A-$NH_2$

AP781 (2-NOA)-NH$(CH_2)_4$CO-A-M-D-R-I-$NH_2$

AP782 (2-NOA)-NH$(CH_2)_4$CO-A-M-D-R-I-G-$NH_2$

AP783 (2-NOA)-NH$(CH_2)_4$CO-A-M-D-R-I-G-A-$NH_2$

AP784 (2-NOA)-G-G-R-I-D-R-NH-$CH_2$-$CH(CH_3)CH_2CH_3$

AP785 (2-NOA)-G-G-R-M-D-R-NH-$CH_2$-$CH(CH_3)CH_2CH_3$

AP786 (2-NA)-G-G-R-M-D-R-NH-$CH_2$-$CH(CH_3)CH_2CH_3$

AP787 (2-NOA)-G-G-R-M-D-R-NH-$CH_2$-$CH(CH_3)_2$

AP788 (2-NA)-G-G-R-M-D-R-NH-$CH_2$-$CH(CH_3)_2$

* AP789 (2-NOA)-NH-$(CH_2)_7$-CO-R-I-D-R-I-$NH_2$

* AP790 (2-NYL)-NH-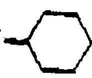-$CH_2$-CO-R-I-D-R-I-$NH_2$

* AP791 (2-NOA)-NH-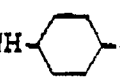-CO-R-I-D-R-I-$NH_2$

* AP792 (2-NOA)-NH-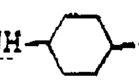-$CH_2$-CO-R-I-D-R-I-$NH_2$

* AP793 (2-NOA)-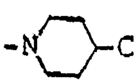-CO-R-I-D-R-I-$NH_2$

* AP794 (2-NYL)-$CH_2$NH-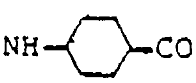-CO-R-I-D-R-I-$NH_2$

* AP795 (2-NYL)-NH$CH_2$-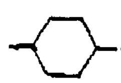-CO-R-I-D-R-I-$NH_2$

* AP796 (2-NL)-$CH_2CH_2NH$-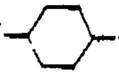-$CH_2$-CO-R-I-D-R-I-$NH_2$

AP797 (2-NYL)-NH-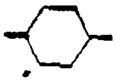-$CH_2$-CO-R-M-D-R-I-$NH_2$

* AP798 (2-NOA)-NH-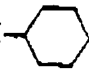-$CH_2$-CO-K-I-D-R-I-$NH_2$

* AP799 (2-NA)-NH-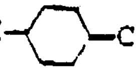-$CH_2CO$-R-I-D-K-I-$NH_2$

* AP800 (2-NA)-NH-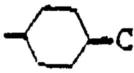-$CH_2$-CO-K-I-D-R-I-$NH_2$

* AP801 (2-NOA)-NH-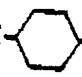-$CH_2$-CO-(Acetyl) Lys-I-D-R-I-$NH_2$

* AP802 (2-NYL)-NH-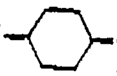-$CH_2$-CO-K-I-D-R-I-$NH_2$

AP803 (2-NYL)-NH-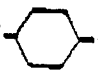-$CH_2$-CO-R-I-D-K-I-$NH_2$

AP804 (2-NYL)-NH-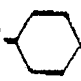-$CH_2$-CO-R-I-D-R-[D-Ile]-$NH_2$

AP805 (2-NYL)-NH-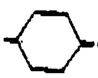-$CH_2$-CO-R-L-D-R-I-$NH_2$

* AP806 (2-NA)-NH-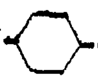-$CH_2CO$-K($CH_2NH$)I-D-R-I-$NH_2$

* AP807 (2-NA)-NH-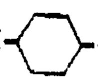-$CH_2$-CO-R-I-D-K($CH_2NH$)I-$NH_2$

* AP808 (2-NOA)-NH-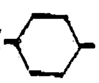-$CH_2CO$-K($CH_2NH$)I-D-R-I-$NH_2$

* AP809 (2-NYL)-NH-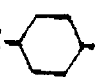-$CH_2$-CO-K($CH_2NH$)I-D-R-I-$NH_2$

* AP810 (2-NOA)-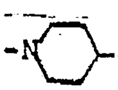-CO-R-I-D-R-NH-(S)-2-methylbutyl

* AP811 (2-NYL)-NH-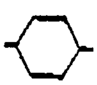-$CH_2CO$-R-I-D-R-NH-(S)-2-methylbutyl

* AP812 (2-NOA)-N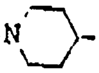-$CH_2$NH-R-I-D-R-NH-(S)-2-methylbutyl

* AP813 (2-NOA)-NH-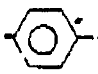-$CH_2$CO-R-I-D-R-NH-(S)-2-methylbutyl

* AP814 (2-NYL)-NH-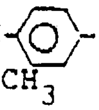-$CH_2$-CO-K($CH_2$NH)I-D-R-$NHCH_2CH(CH_3)CH_2CH_3$

AP815 (2-NOA)-NH-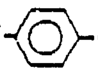-$CH_2$CO-Orn-I-D-R-I-$NH_2$

AP816 (2-NOA)-NH-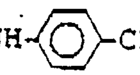-$CH_2$($CH_2$NH)R-I-D-R-I-$NH_2$

AP817 (2-ClPA)-NH-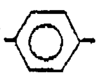-$CH_2$CO-R-I-D-R-I-$NH_2$

AP818 (2-BrPA)-NH-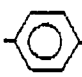-$CH_2$CO-R-I-D-R-I-$NH_2$

AP819 (2-BrAF)-NH-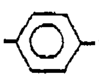-$CH_2$CO-R-I-D-R-I-$NH_2$

Figure 3

Sheet 1 of 2

* AP105 G-G-R-I-D-R-I-G-A-A-$NH_2$

* AP106 G-R-I-D-R-I-G-A-A-$NH_2$

* AP107 R-I-D-R-I-G-A-A-$NH_2$

* AP120 Acetyl-G-G-R-I-D-R-I-$NH_2$

AP121 G-R-I-D-R-I-$NH_2$

AP123 G-G-R-I-D-R-I-G-$NH_2$

AP124 G-R-I-D-R-I-G-$NH_2$

AP125 R-I-D-R-I-G-$NH_2$

AP127 G-G-R-I-D-R-I-G-A-$NH_2$

AP128 G-R-I-D-R-I-G-A-$NH_2$

AP129 R-I-D-R-I-G-A-$NH_2$

AP291 Acetyl-G-G-R-M-D-R-I-$NH_2$

AP292 G-R-M-D-R-I-$NH_2$

AP294 G-G-R-M-D-R-I-G-$NH_2$

AP295 G-R-M-D-R-I-G-$NH_2$

AP296 R-M-D-R-I-G-$NH_2$

AP298 G-G-R-M-D-R-I-G-A-$NH_2$

| | |
|---|---|
| AP299 | G-R-M-D-R-I-G-A-$NH_2$ |
| AP300 | R-M-D-R-I-G-A-$NH_2$ |
| * AP301 | A-F-G-G-$R^{\dagger}$-I-D-R-I-G-A-$NH_2$ |
| * AP303 | A-F-G-G-R-I-$D^{\dagger}$-R-I-G-A-$NH_2$ |
| AP512 | (2-NA)-G-G-L-I-D-R-I-$NH_2$ |
| AP514 | (2-NA)-G-G-Nle-I-D-R-I-$NH_2$ |
| AP515 | (2-NA)-G-G-R-MSO-D-R-I-$NH_2$ |
| AP516 | (2-NA)-G-G-R-I-D-S-I-$NH_2$ |
| AP519 | (2-NA)-G-G-R-I-D-R-P-$NH_2$ |
| AP521 | (2-NA)-G-G-R-F-D-R-I-$NH_2$ |
| AP537 | (2-NA)-G-G-R-I-D-R-$F^{\dagger}$-$NH_2$ |
| AP558 | (2-NA)-G-G-R-I-(beta-$Asp^{\dagger}$)-R-I-$NH_2$ |
| AP559 | (2-NA)-G-G-R-I-(beta-Asp)-R-I-$NH_2$ |
| AP575 | (2-NA)-G-G-R-Phg-D-R-I-$NH_2$ |
| AP595 | (2-NA)-G-G-R-I-D-R-Phg-$NH_2$ |